# EUROPEAN PATENT SPECIFICATION

(11) **EP 2 440 225 B1**
(45) Date of publication and mention of the grant of the patent: **18.10.2017**
(21) Application number: 10725159.7
(22) Date of filing: 11.06.2010
(51) Int. Cl.: A61K 38/00

(54) **NOVEL APPLICATIONS OF HIP/PAP OR DERIVATIVES THEREOF**
NEUARTIGE ANWENDUNGEN VON HIP/PAP UND DERIVATE DAVON
NOUVELLES APPLICATIONS DE HIP/PAP OU DÉRIVÉS ASSOCIÉS

(30) Priority: 11.06.2009 EP 09290437
(43) Date of publication of application: 18.04.2012
(73) Proprietor: Alfact Innovation, 75001 Paris (FR); Brechot, Christian, 75015 Paris (FR)
(72) Inventor: FAIVRE, Jamila, F-75016 Paris (FR); GRESSENS, Pierre, F-95470 Saint-Witz (FR); AMOUYAL, Gilles, F-75016 Paris (FR); AMOUYAL, Paul, F-92310 Sevres (FR); BRECHOT, Christian, F-75015 Paris (FR); ROUGIER, Elodie, F-87600 Cheronnac (FR)
(74) Representative: Nony
(86) International application number: PCT/EP2010/058270
(87) International publication number: WO 2010/142800

(56) References cited:
- EP-A- 1 488 798
- WO-A-01/23426
- WO-A-91/16428
- WO-A2-02/059315
- WO-A2-03/016475
- US-A1- 2005 277 593
- K. Namikawa: "Pancreatitis-Associated Protein-III Is a Novel Macrophage Chemoattractant Implicated in Nerve Regeneration", Journal of Neuroscience, vol. 26, no. 28, 12 July 2006 (2006-07-12) , pages 7460-7467, XP055124677, ISSN: 0270-6474, DOI: 10.1523/JNEUROSCI.0023-06.2006

## Description

### FIELD OF THE INVENTION

This invention relates to the field of neurology, and more particularly to the field of neonatal brain injury.

### BACKGROUND OF THE INVENTION

Neonatal brain injury constitutes a specific field of medical investigations, since processes involved in neonatal neuronal injury are distinct from those leading to neuronal injury in adult patients.

In spite of improved obstetric and neonatal care, two groups of newborn infants are still at risk of developing perinatal brain injury with permanent neurological impairment, namely the preterm infant with predominantly white-mater damage and the term asphyxiated infant with hypoxic-ischemic brain injury. In addition to hypoxia-ischemia, an enhanced release of glutamate leading to excitotoxic cell death and the production of reactive oxygen species (ROS) are important in the brain injury process in both these groups.

Neonatal asphyxia, also known as hypoxia-ischemia (HI), is a condition arising from the inadequate intake of oxygen in an infant during preterm development, labour, delivery, or the immediate postnatal period. Neonatal asphyxia remains a major cause of chronic neurological morbidity and acute mortality in the newborn and commonly leads to hypoxic-ischemic encephalopathy. The existing studies have shown that neonatal hypoxia for a time as short as six minutes can lead to permanent neurological damage. About 14.6% of all deaths at birth are caused by neonatal hypoxia. In the western world, about 0.9% of newborns suffer from neonatal hypoxia. About 15-20% die, and among the survivors, 25% are severely handicapped due to long-term complications such as mental retardation, cerebral palsy, spasticity, learning difficulties or epilepsy. Furthermore, it is increasingly recognized that children with rather mild hypoxia, who seem initially to recover without complications, have behavioural problems in childhood, which can be tracked back to this neonatal insult.

Generally, the initiation and development of injury to the neonatal brain are complex processes, with multiple contributing mechanisms and pathways resulting in both early and delayed injury. Studies have focused on various aspects of these processes, including oxidative stress, inflammation and excitotoxicity, which are widely different in the mature or immature brain (Gonzalez et al., 2008, Pharmacology and Therapeutics, Vol. 120: 43-53).

Currently, there are no approved therapies for the brain damage associated with neonatal HI beyond the supportive therapies and interventions provided as part of intensive care. However, there is a lot of promising avenues of research to improve intervention and discover new strategies in the wake of the therapies that have been tested for clinical applications, and failed. Examples of failed therapies include supplemental glucose during resuscitation, hyperventilation, midazolam, barbiturates, dexamethasone and other glucocorticoids, calcium-channel blockers, magnesium sulfate, indomethacin, superoxide dismutase/catalase conjugated to polyethylene glycol, MK-801, detromethorphan and caspase inhibitors. The management of neonatal cerebral HI includes supportive care to provide adequate ventilation, meticulous fluid management, avoidance of hypotension and hypoglycemia, and treatment of seizures. Indeed, it has been hypothesized that improving the manner in which infants are resuscitated post-HI may be a crucial first step forward for improving outcome and reducing disability. The concentration of oxygen used during resuscitation may affect brain damage after HI. Hyperbaric oxygen has been tried in neonates with HI and in rodent models of focal ischemic stroke with some success. Further studies are required to clarify the role of oxygen in neonates with stroke, who are also at risk for 'oxygen free radical disease.

Beyond improving intensive care, many studies have been performed to identify new treatment options and potential interventions to ameliorate the process of ongoing brain injury. Briefly, various strategies have been explored, including testing antioxidants, anti-inflammatory agents, cell death inhibitors, growth factors, stem cell therapy or hypothermia. A variety of anti-inflammatory compounds have been investigated. Several interventions, such as allopurinol/oxypurinol, desferoxamine and melatonin, attempt to reduce HI-induced cerebral damage resulting from free radicals have failed to raise clinical interest because of their side-effect profiles and some failed trials. Recent promising therapies include erythropoietin and *N-*acetylcysteine. Erythropoietin is an antioxidant that possesses antiapoptotic, antinitrosative and angiogenic properties. Erythropoietin has also been reported to promote neuroprotection and neurogenesis through the alteration of cell-fate decisions, and to improve functional outcome after stroke in immature rats. *N*-acetylcysteine, a well-known antioxidant and free-radical-scavenging agent, can cross the placenta and the blood brain barrier (BBB) and has a good safety profile during pregnancy. *N*-acetylcysteine has been shown to reduce oxidative stress, restore intracellular glutathione levels, scavenge oxygen free radicals, improve cellular redox potential and reduce apoptosis and inflammation of the neonatal brain, in addition to the improved cardio-renal recovery observed after asphyxia. Hydrogen therapy might also represent a treatment strategy to explore. Additionally, attempts to reduce brain injury in neonatal hypoxic-ischemic rats by intra-cerebroventicular injection of neural stem/progenitor cells together with chondroitinase ABC have been made (Sato et al., 2008, Reprod Sci., Vol. 15(n°6) : 613-620).

Among the brain traumas causing great medical concerns, traumatic brain injury (TBI) occurs when an outside force injures the brain. TBI is a major cause of death and disability worldwide, especially in children and young adults. TBI is defined as damage to the brain resulting from external mechanical force, such as rapid acceleration or deceleration, impact, blast waves, or penetration by a projectile. Brain traumas cause secondary injury, which includes alterations in the cerebral blood flow and the pressure within the skull. TBI can cause a host of physical, cognitive, emotional and behavioural effects. TBI outcome can range from complete recovery to permanent disability or death. The 20th century has seen critical developments in diagnosis and treatment which have decreased death rates and improved outcome. These include imaging techniques such as computed tomography and magnetic resonance imaging. People affected with a TBI shall be urgently subjected to an emergency medical treatment within the so-called "golden hour" following the injury. People with moderate to severe injuries are likely to receive treatment in an intensive care unit followed by a neurosurgical ward.. Treatment depends on the recovery stage of the patient. In the acute stage, the primary aim is to stabilize the patient and focus on preventing further injury, since little can be done to reverse the initial damage caused by trauma. The primary medical concerns are ensuring proper oxygen supply, maintaining adequate cerebral blood flow, and controlling raised intracranial pressure. Research is presently performed for designing potential medical treatments that may help in neuroprotection of the patient. However, clinical trials to test agents that could halt brain cell injury have largely met with failure.

For example, interest existed in hypothermia, cooling the injured brain to limit TBI damage, but clinical trials showed that it is not useful in the treatment of TBI. These failures could be due to factors including faults in the trials' design or in the insufficiency of a single agent to prevent the array of injury processes involved in secondary injury

Hyperbaric oxygen therapy (HBO) has been evaluated as an adjunctive treatment following TBI concluding a Cochrane review that its use could not be justified HBO for TBI has remained controversial as studies have looked for improvement mechanisms, and further evidence shows that it may have potential as a treatment.

The previous remarks illustrate that, to date, medical advances are highly needed for treating patients affected with a traumatic brain injury.

There is a need in the art for identifying further compounds that may effectively treat neonatal brain injury.

There is also a need in the art for identifying compounds that are useful for treating traumatic brain injury (TBI) which causes neuronal cell death, for which very few methods of treatment are presently available.

There is also a need in the art for identifying compounds that are useful for treating cerebellum diseases, which encompasses cerebellum genetic diseases, as well as environmental cerebellum diseases.

There is also a need in the art for identifying compounds that are useful for treating Alzheimer's disease as well as mind cognitive impairment preceding Alzheimer's disease specific symptoms.

### SUMMARY OF THE INVENTION

The instant invention provides further compounds for preventing or treating various types of brain injury.

This invention concerns in particular HIP/PAP protein or a protein derivative thereof for use in preventing or treating a neonatal brain injury, which includes a neonatal brain injury caused by a brain hypoxia, wherein the "HIP/PAP" protein comprises an amino acid sequence having at least 90% amino acid identity with a polypeptide selected from the group consisting of the polypeptides of SEQ ID NO 1 to 3,
wherein the protein derivative thereof is selected from the group consisting of a biologically active portion of a "HIP/PAP" protein and "HIP/PAP" chimeric or fusion proteins,
and wherein the said neonatal brain injury encompasses a pathological state selected from the group consisting of foetal hypoxemia, perinatal hypoxemia, foetal ischemia, perinatal ischemia, and an excess release of excitatory neurotransmitters

This invention also relates to HIP/PAP protein or a protein derivative thereof for use in preventing or treating a neonatal brain injury consisting of a cerebrovascular accident, which encompasses stroke, and wherein stroke is preferably selected from the group consisting of ischemic stroke and hemorrhagic stroke.

### BRIEF DESCIPTION OF THE FIGURES

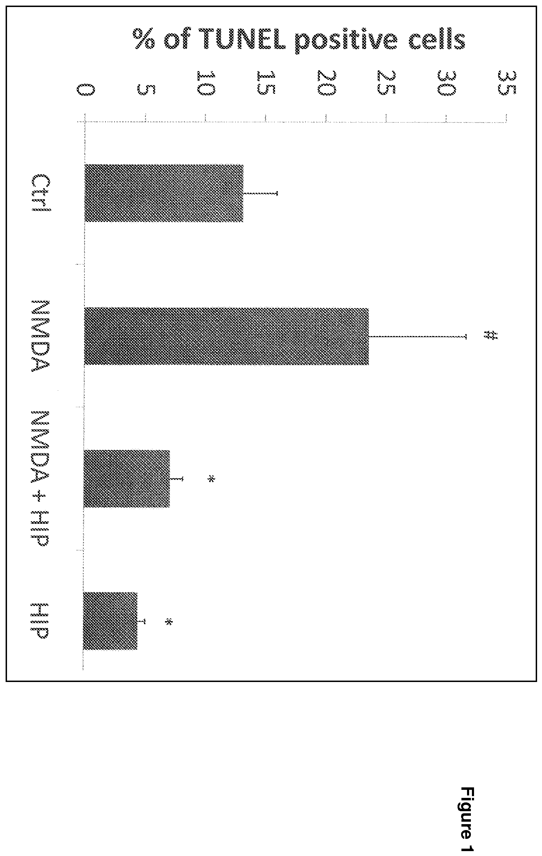

**Figure 1** illustrates the protective effect of HIP/PAP against cell apoptosis of cortical neuronal cells from GAP 43 +/+ mice. Ordinate : percentage of TUNEL positive cells; Abscissa : from left to right, cells incubated with (1) Control culture medium, (2) NMDA, (3) NMDA and HIP/PAP, and (4) NMDA alone. '#" : significant as compared to Control; "*" : significant as compared to NMDA.

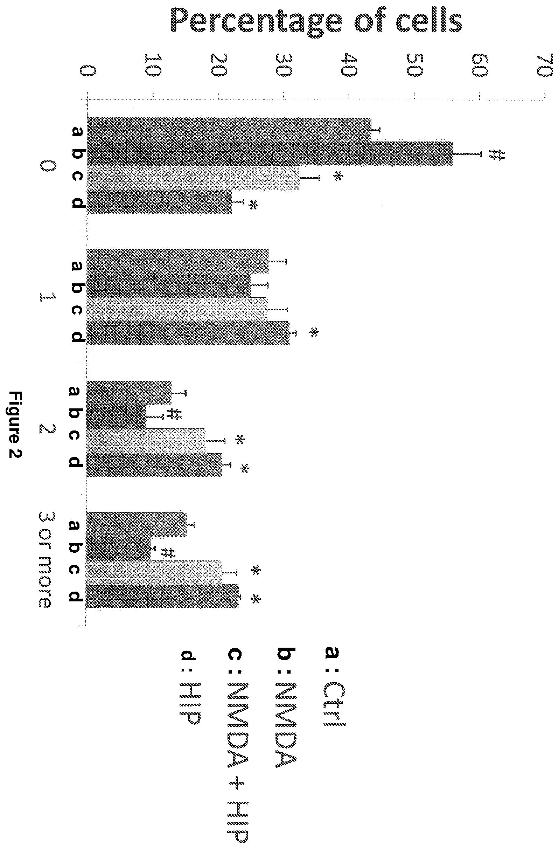

**Figure 2** illustrates the effect of HIP/PAP on the number of primary neurites in cortical neuronal cells from GAP 43 +/+ mice. Ordinate : percentage of cells having a specific number of primary neurites. Abscissa : four sets of bars, from left to right, respectively : (1) cells with 0 primary neurite, (2) cells with 1 primary neurite, (3) cells with 2 primary neurites, (4) cells with 3 primary neurites or more; each set of bars illustrates the results obtained with, from left to right : (a) Control culture medium, (b) NMDA, (c) NMDA and HIP/PAP, and (d) HIP/PAP alone; . '#" : significant as compared to Control; "*" : significant as compared to NMDA.

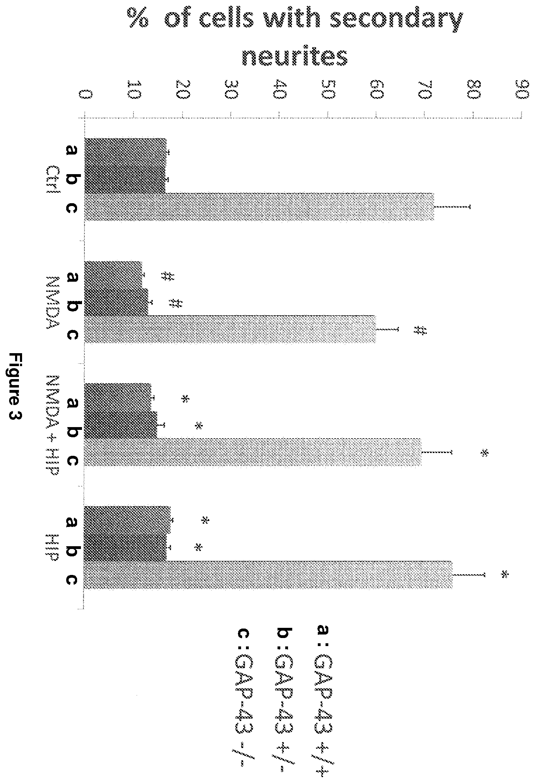

**Figure 3** illustrates the effect of HIP/PAP on the number of secondary neurites in mice cortical neuronal cells. Ordinate : percentage of cells having secondary neurites. Abscissa : four sets of bars, from left to right, respectively : (1) Control culture medium, (2) NMDA, (3) NMDA and HIP/PAP, and (4) HIP/PAP alone; each set of bars illustrates the results obtained with mice cortical cells originating from, from left to right: (a) GAP 43+/+ mice, (b) GAP 43 +/- mice and (c) GAP 43 -/- mice; . '#" : significant as compared to Control; "*" : significant as compared to NMDA.

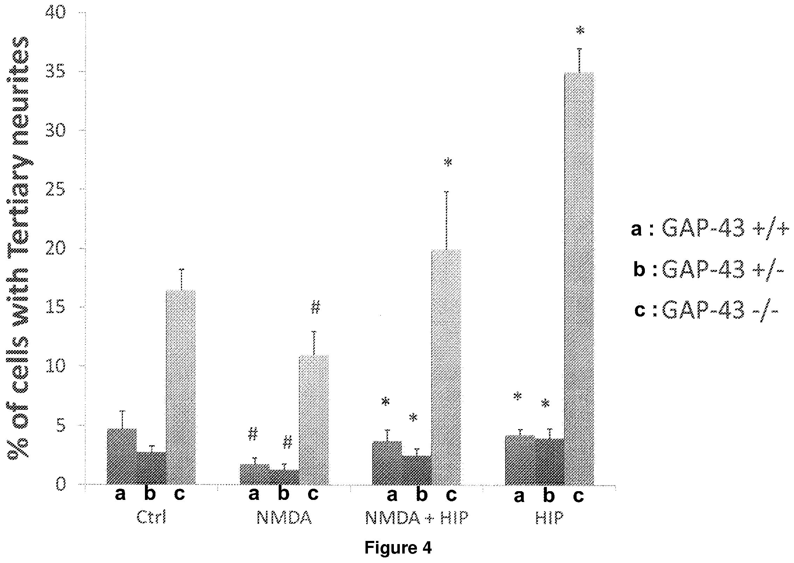

**Figure 4** illustrates the effect of HIP/PAP on the number of tertiary neurites in mice cortical neuronal. Ordinate : percentage of cells having tertiary neurites. Abscissa : four sets of bars, from left to right, respectively : (1) Control culture medium, (2) NMDA, (3) NMDA and HIP/PAP, and (4) HIP/PAP alone; each set of bars illustrates the results obtained with mice cortical cells originating from, from left to right : (a) GAP 43+/+ mice, (b) GAP 43 +/- mice and (c) GAP 43 -/mice; . '#": significant as compared to Control; "*" : significant as compared to NMDA.

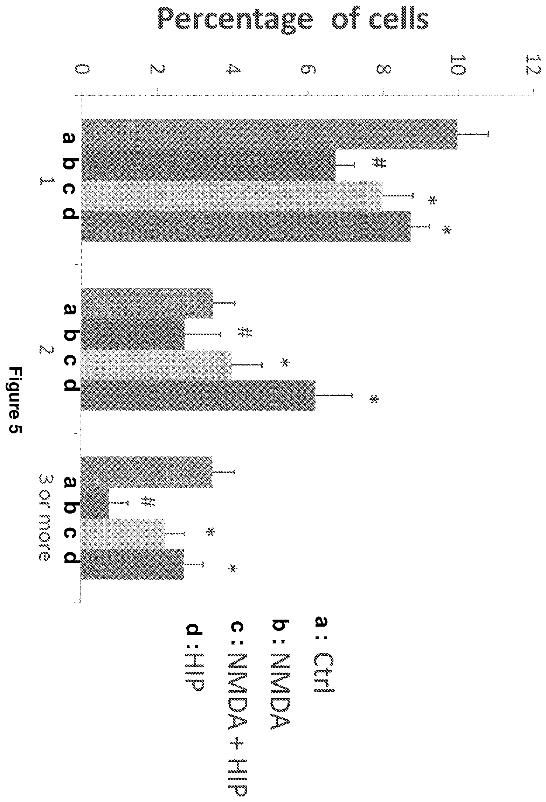

**Figure 5** illustrates the effect of HIP/PAP on the number of branch points in cortical neuronal cells from GAP 43 +/+ mice. Ordinate : percentage of cells having a specific number of branch points. Abscissa : three sets of bars, from left to right, respectively : (1) cells with 1 branch point, (2) cells with 2 branch points, (3) cells with 3 branch points or more; each set of bars illustrates the results obtained with, from left to right : (a) Control culture medium, (b) NMDA, (c) NMDA and HIP/PAP, and (d) HIP/PAP alone; . '#" : significant as compared to Control; "*" : significant as compared to NMDA.

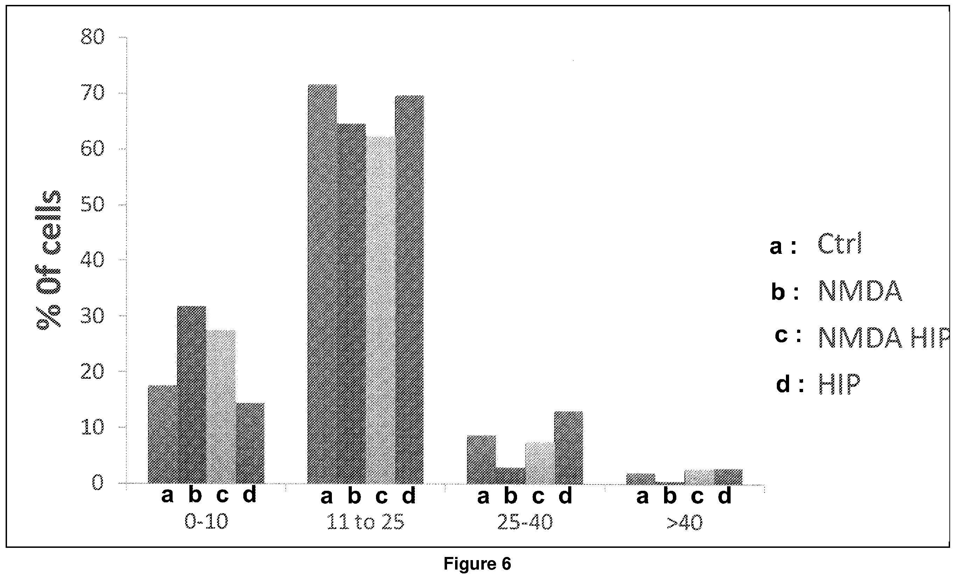

**Figure 6** illustrates the effect of HIP/PAP on the neurite length of cortical neuronal cells from GAP 43 +/+ mice. Ordinate : percentage of cells having a specific neurite length range. Abscissa : four sets of bars, from left to right, respectively : (1) cells with a neurite length ranging from 0 to 10 µm, (2) cells with a neurite length ranging from 11 to 25 µm, (3) cells with a neurite length ranging from 25 to 40 µm and (4) cells with a neurite length of more than 40 µm; each set of bars illustrates the results obtained with, from left to right : (a) Control culture medium, (b) NMDA, (c) NMDA and HIP/PAP, and (d) HIP/PAP alone; . '#" : significant as compared to Control; "*" : significant as compared to NMDA.

### DETAILED DESCRIPTION OF THE INVENTION

It has been found herein that the HIP/PAP protein or a protein derivative thereof is able to protect against neuronal cell death, especially against neuronal cell death that occurs in neonatal brain injury.

One is reminded herein that the HIP/PAP protein was previously known in the art as a mitogenic factor for hepatocytes. HIP/PAP (Reg IIIa) gene was also previously known for being up-regulated in a mouse experimental model of an inflammatory brain disease, namely experimental autoimmune encephalomyelitis (EAE), and HIP/PAP protein was shown to potentially exert a preventive effect on mouse EAE. These preliminary data had led previous authors to allege a beneficial effect of Reg III protein for treating a very large number of inflammatory or neurological diseases of the adult patients, including multiple sclerosis, optic neuritis, neuromyelytis optica, adrenoleukodistrophy, adrenomyeloneuropathy, spinal cord injury, amyotrophic lateral sclerosis, inflammatory bowel disease or also sepsis (See United States patent application n° US 2005/0277593). It is reminded herein that the EAE consists of an animal model of brain inflammation mimicking an inflammatory demyelinating disease of the central nervous system, like multiple sclerosis and acute disseminated encephalomyelitis (ADEM). EAE is also the prototype for T-cell mediated autoimmune disease in general. It is also recalled that myelin consists of an outgrowth of glial cells and that Scwann cells supply the myelin for peripheral neurons, whereas oligodendrocytes, specifically of the intrafascicular type, myelinate the axons of the central nervous system. Demyelinating diseases essentially encompass multiple sclerosis, acute disseminated encephalomyelitis, transverse myelitis, chronic inflammatory demyelinating polyneuropathy, Guillain-Barré Syndrome, central pontine myelinosis, Tay-Sachs disease, as well as inherited demyelinating diseases such as leucodistrophy and Charcot Marie Tooth. There is no need to say that diseases like sepsis, amyotrophic lateral sclerosis, spinal cord injury, inflammatory bowel disease, Alzheimer's disease, dementia, vasculitis or ischemic insult do not consist of diseases that are initially caused by demyelination. Consequently, the prevention or therapy of these latter diseases by a substance that would inhibit EAE symptoms is devoid of any technical credibility for the one skilled in the art.

It was also shown in the art that there exists a correlation between allelic polymorphisms in the genomic sequence of the HIP/PAP gene and the occurrence of multiple sclerosis (See PCT application n° WO 2007/071437). Finally, it was also shown in the art that Reg family members would be mediators among injured neurons and glial cells and would potentially play a role during nerve regeneration (See Namikawa et al., 2005, Biochem Biophys Res Commun, Vol. 332 (n° 1) : 126-134).

It has been found according to the invention that the HIP/PAP protein, or a protein derivative thereof, possesses neuroprotective properties during a brain injury. It has been found that the HIP/PAP protein, or a protein derivative thereof, possesses neuroprotective properties during neonatal brain injury, thus during a brain damage occurring in the early development which brain damage is completely distinct from the brain injury happening to the developed brain of the adults.

More precisely, it has been found herein that HIP/PAP possesses *in vitro* and *in vivo* neuroprotective effects in experimental models of neonatal brain injury.

This invention relates to the use of the HIP/PAP protein or a protein derivative thereof for protecting neuronal cells from cell apoptosis or cell death.

This invention also relates to the HIP/PAP protein, or a protein derivative thereof, for use for preventing or treating neonate brain injury.

Generally, when it is specified herein uses of the HIP/PAP protein or a protein derivative thereof for preventing or treating pathological states, including traumas, diseases and disorders, this also means aspects of the invention relating to the HIP/PAP protein or a protein derivative thereof for use in preventing or treating the said pathological states.

Generally, when it is specified herein uses of the HIP/PAP protein or a protein derivative thereof for preventing or treating pathological states, including traumas, diseases and disorders, this also means aspects of the invention relating to the use of the HIP/PAP protein or a protein derivative thereof for manufacturing a medicament for preventing or treating the said pathological states.

Generally, when it is specified herein uses of the HIP/PAP protein or a protein derivative thereof for preventing or treating pathological states, including traumas, diseases and disorders, this also means aspects of the invention relating to methods for preventing or treating the said pathological states comprising a step of administering to a patient in need thereof the HIP/PAP protein or a protein derivative thereof.

As used herein, a traumatic brain injury is medically determined and classified according to the Glascow Coma Scale (GCS), according to which a person's level of consciousness is graded on a scale of 3 to 15 based on verbal, motor and eye-opening reactions to stimuli (See Marion DW (1999). "Introduction". in Marion DW. Traumatic Brain Injury. Stuttgart: Thieme).

The present invention relates to the use of the HIP/PAP protein or a protein derivative thereof for preventing or treating a neonatal brain injury, including a neonatal brain injury caused by a brain hypoxia.

It thus relates to the use of the HIP/PAP protein or a protein derivative thereof for manufacturing a medicament for preventing or treating a neonatal brain injury, including a neonatal brain injury caused by a brain hypoxia.

This disclosure pertains to methods for preventing or treating a neonatal brain injury, and especially in experimental models of neonatal brain injury, wherein the said method comprises a step of administering, to the patient in need thereof, a suitable amount of a HIP/PAP protein or of a protein derivative thereof.

This disclosure also provides methods for preventing or treating neonate, traumatic brain injury (TBI), wherein the said method comprises a step of administering, to the patient in need thereof, a suitable amount of a HIP/PAP protein or of a protein derivative thereof.

It also relates to a method for preventing or treating a neonatal brain injury, including a neonatal brain injury caused by a brain hypoxia, wherein the said method comprises a step of administering, to the patient in need thereof, a suitable amount of a HIP/PAP protein or of a protein derivative thereof.

This disclosure also relates to the use of the HIP/PAP protein or a protein derivative thereof for preventing or treating neonate, traumatic brain injury (TBI).

This disclosure encompasses the use of the HIP/PAP protein or a protein derivative thereof for preventing or treating neonatal brain injury.

This disclosure also relates to the use of the HIP/PAP protein or a protein derivative thereof for manufacturing a medicament for preventing or treating foetal, neonate, child and adult traumatic brain injury (TBI).

It thus pertains to the use of the HIP/PAP protein or a protein derivative thereof for manufacturing a medicament for preventing or treating neonatal brain injury.

It also relates to a method for preventing or treating a neonatal brain injury, wherein the said method comprises a step of administering, to the patient in need thereof, a suitable amount of a HIP/PAP protein or of a protein derivative thereof.

In most embodiments of the preventive or therapeutic methods described in the present specification, the said HIP/PAP protein or the said protein derivative thereof is combined with one or more pharmaceutically acceptable excipients, to form a pharmaceutical composition.

Pharmaceutically acceptable excipients preferably consist of those which are agreed or listed in the US Pharmacopeia or in the European Pharmacopeia.

This invention provides novel clues to the neuronal protection against neonatal brain injury/trauma/degeneration by providing new protective compositions.

By "HIP/PAP protein", it is intended herein a protein comprising the amino acid sequence of SEQ ID N°1. Specific embodiments of a HIP/PAP protein encompass proteins comprising an amino acid sequence selected from the group consisting of SEQ ID N° 2 and SEQ ID N° 3. The HIP/PAP protein of SEQ ID N° 3 consists of the HIP/PAP protein that is more specifically illustrated in the examples herein, where it may also be termed "ALF5755". As illustrated in the examples herein, the HIP/PAP protein of SEQ ID N° 3 may be recombinantly produced in *E. coli.* The HIP/PAP protein of SEQ ID N° 3 comprises a N-terminal propeptide portion of 12 amino acids that is cleaved *in vivo* for releasing the biologically active portion of the said HIP/PAP protein. The said biologically active portion of the said HIP/PAP protein is located from the Isoleucine residue in position 13 to the aspartic acid residue located in position 150 of SEQ ID N° 3. Similarly; the said biologically active portion of the said HIP/PAP protein is located from the tryptophan residue in position 12 to the aspartic acid residue located in position 149 of SEQ ID N° 2.

Preferably, any embodiment of a HIP/PAP protein, as well as any embodiment of a derivative thereof, consists of a recombinant protein, e.g. a protein that is recombinantly produced in bacterial or animal cells, including insect cells and mammal cells.

The inventors have shown that HIP/PAP protein significantly reduces neuron death or apoptosis in a neonatal mouse model of excitotoxic brain lesions.

The invention also provides therapeutic tools against the oxidative stress, which is a major factor of neuronal abnormalities and degeneration, through the antioxidant power of HIP/PAP, as further detailed below. Moreover, and interestingly, the effect of HIP/PAP protein is also observed when it is administered at the periphery, instead of being directly delivered to the CNS. Finally, protective effects of HIP/PAP protein encompass prevention of neuronal death and induction of neuronal plasticity.

This patent also relates to the use of the HIP/PAP protein or a protein derivative thereof for modulating the structural remodelling or plasticity of neuronal cells.

As it is shown in the examples herein, a HIP/PAP protein prevents *in vitro* the N-methyl-D-aspartate (NMDA)-induced neuronal cell death in mice neuronal primary cultures. It is also shown herein that a HIP/PAP protein rescues *in vitro* the N-methyl-D-aspartate (NMDA)-induced neuronal cell death in primary mice neuronal cultures.

Also, it is shown that a HIP/PAP protein prevents *in vivo* the excitotoxic lesions caused by the glutamanergic analogue ibotenate in the developing mouse brain.

Additionally, the results presented in the examples disclose that a HIP/PAP protein possesses an *in vivo* rescuing effect on the excitotoxic lesions caused by the glutamanergic analogue ibotenate in the developing mouse brain.

Still further, it has been found according to the invention that the preventive and curative *in vivo* effects of the HIP/PAP protein on neonatal brain injury might be the result of pleiotropic activities of HIP/PAP, i.e. of the involvement of HIP/PAP in a plurality of neuronal rescuing physiological pathways.

As an illustration of the pleiotropic activities of HIP/PAP in the prevention or the treatment of neonatal brain injury, it is also shown herein that HIP/PAP effects neuroprotection against neonatal neuronal cell death induced by a reactive oxygen species like hydrogen peroxide (H₂O₂).

As a further illustration of the pleiotropic activities of HIP/PAP in the prevention or the treatment of neonatal brain injury, it is also shown herein that, in the *in vitro* and *in vivo* models of neonatal brain injury, HIP/PAP induces changes in the production, phosphorylation and distribution of GAP-43 or phosphorylated GAP-43, which is a protein known in the art to be involved in axonal growth and neuronal plasticity.

Notably, HIP/PAP induces a fascicular distribution of GAP-43-labelled neuronal processes.

Also, HIP/PAP induces an increased expression of the phosphorylated form of the GAP-43 protein, i.e. an increased expression of the form of GAP-43 which is phosphorylated at the serine residue located at position 41 (S41) of the GAP-43 amino acid sequence. HIP/PAP rather induces a decrease in the total cellular amount of GAP-43 while, at the same time, it induces an increase in the phospho(S41)-GAP-43.

Without wishing to be bound by any particular theory, the inventors believe that the HIP/PAP biological effects on GAP-43 are important for preventing or treating a brain injury caused by a brain hypoxia, and especially for preventing or treating a neonatal brain injury. In this context, the inventors believe that the effects of HIP/PAP on GAP-43 production and metabolism moderate the consequences of the death or of the apoptosis of certain neuronal cells.

Nevertheless, it has been shown in the examples herein that the protective effect of HIP/PAP against neuronal cell death or neuronal cell apoptosis is not compulsorily mediated by an action of HIP/PAP on GAP43, since the said protective effect of HIP/PAP is retained in mammals which do not produce the GAP43 protein (GAP43 -/-).

Additionally, it is shown in the examples herein that HIP/PAP induces or increases neuroplasticity, even in mammals which do not produce GAP43 (GAP43 -/-).

The increased neuronal plasticity that is caused by HIP/PAP is illustrated in the examples herein through the ability of HIP/PAP to generate an increased number of neurites, as well as to generate neurites having a high complexity (presence of primary, secondary and tertiary neurites, as well as of neurites having an increased number of branch points).

The increased neuronal plasticity that is caused by HIP/PAP is also illustrated in the examples herein through the ability of HIP/PAP to reverse the deleterious effect of toxic agents, like NMDA, on neurite growth.

As used herein, the term "neuroplasticity", which may be used interchangeably with the term "brain plasticity" herein, refers to the brain ability to change its structure and function during maturation, learning, environmental challenges or pathology, including traumatic events like traumatic brain injury (TBI) which may be also called intracranial injury.

The increased neuroplasticity that is caused by HIP/PAP finds its therapeutic usefulness notably in methods for treating patients who have undergone an adverse event causing an interruption in the blood supply to brain, which includes traumatic brain injury and stroke. Indeed, plastic phenomena occurring in the cerebral cortex and in subcortical structures after ischemic injuries are well documented at the synaptic, cellular, and network level. Previous works show that neuroplasticity play a key role during neurorehabilitation of post-ischemic brain, in human stroke survivors.

The above experimental results support the usefulness of a HIP/PAP protein, both *in vitro* and *in vivo,* for modulating, enhancing or improving neuronal growth or neuronal plasticity, the said modulation or the said improvement of neuronal growth or neuronal plasticity being either mediated by Gap-43 or not being mediated by Gap-43.

Furthermore, as described in the examples herein, a HIP/PAP protein prevents both *in vitro* excitotoxicity-induced cell death and oxidative cell death in primary culture of mice cerebellum granular cells. These results support the usefulness of a HIP/PAP protein for preventing and treating cerebellar conditions such as spinocerebellar ataxia.

These experimental results also support the neuroprotective activity of a HIP/PAP protein towards a plurality of neuronal cell types, in particular those present in cortex and cerebellum.

As used herein, a brain hypoxia encompasses situations wherein a brain injury is caused by a reduced oxygen supply to the brain, and especially to neuronal cells, and wherein brain hypoxia causes neuronal cell death or apoptosis. Brain hypoxia is considered as occurring when blood vessels are obstructed and/or when there occurs any other cause of oxygen depletion in the brain tissues, especially asphyxia.

As used herein, a "neonatal" brain injury encompasses any injury that is caused to the brain of foetus (also termed foetal brain injury), pre-term neonates, term and post-term neonates, as well as children being less than one year old. Thus, neonatal brain injury encompasses what is conventionally termed « perinatal » brain injury which occurs immediately before and after delivery, as well as what is conventionally termed neonatal brain injury, which occurs during the perinatal period up through four weeks of age. Neonatal brain injury includes stroke, birth trauma, metabolic or genetic disorders, status epilepticus, and a variety of asphyxial events resulting in hypoxia and ischemia (HI). HI often leads to periventricular white matter injury in premature infants, while term infants develop cortical/subcortical lesions. Generally, it is admitted in the art that neonatal brain injury is recognised on the basis of a unique form of encephalopathy that evolves from lethargy to hyperexcitability to stupor during the first three days of life. A review of the available methods of diagnosing neonatal brain injury is disclosed by Ferriero, to which the one skilled in the art may refer, the entire content of which is herein enclosed (Ferriero, 204, New England Journal of Medicine, Vol. 351 (n° 19): 1985-1995)

In foetal life, neonatal brain injury may be caused (i) by hypoxic-ischemic disturbances due to hypoperfusion induced by low arterial partial pressure of oxygen or (ii) by severe hypoxemia that leads to myocardial dysfunction with subsequent cerebral hypoperfusion leading to neuronal ischemia.

Neonatal brain injury encompasses foetal hypoxemia that is usually the result of placental insufficiency.

Neonatal brain injury also encompasses perinatal hypoxemia, which is a post-natal hypoxemia resulting generally from either respiratory or cardiac insufficiency, alone or in combination.

Neonatal brain injury encompasses foetal ischemia.

Neonatal brain injury encompasses perinatal ischemia, which is a post-natal ischemia, which may be due to a severe cardiac contractile dysfunction that leads to cerebral hypoperfusion and loss of cerebrovascular regulation. Circulatory insufficiency may result from severe prenatal or post-natal haemorrhage or neonatal sepsis.

Neonatal brain injury also encompasses the injury which is caused by pressure-passive cerebral circulation, where autoregulation of cerebral circulation is impaired and hypoxemia is induced.

Neonatal brain injury also encompasses neonatal hypoxia that is due to an underperfusion that affects the depths of the sulci and generates an increased susceptibility to hypotensive insults.

Indeed, neonatal injury encompasses excess release or excitatory neurotransmitters during neonatal brain hypoxia.

According to the present invention, a HIP/PAP protein or a protein derivative thereof may be used for preventing or treating a neonatal brain injury with a grading classification ranging from stage 1 to stage 3, according to the conventional grading system of Sarnat used for classifying hypoxic-ischemic encephalopathy (HIE).

Stage 1 of the Sarnat grading system relies on the finding of an early syndrome characterized by hyperalertness and sympathetic activation, excessive reaction to stimuli, weak suck with normal tone, and electroencephalogram (EEG) findings. This stage lasts less than 24 hours, and patients who remain in stage 1 have normal neurologic outcomes.

Patients progressing to stage 2 of the Sarnat grading system have mild hypotonia, are lethargic or obtunded (e.g., have a delayed and incomplete response to sensory stimuli), clinical seizures, parasympathetic activation with myosis (even in dim light), slowed heart rate (<120 bpm), increased peristalsis, and copious secretions. Early on, EEG shows relatively low-voltage amplitude (<25 µV in the slow theta and delta range). On the second day, the EEG demonstrates a bursting pattern during wakefulness or obtundation (which worsens during sleep) and multifocal, low-frequency (1- to 1.5-Hz) EEG seizures with a central or temporal predominance. If the EEG recovers within 5 days, it becomes completely normal again. If the recovery takes more than 5 days, low-amplitude slowing is noted. Stage 2 lasts 2-14 days. Clinical and EEG recovery within 5 days is associated with a good prognosis. Periodic EEG may indicate a poor prognosis if interburst intervals are totally isoelectric or if the bursting frequency is less than every 6 seconds, with a bursting pattern (every 3-6 seconds) lasting more than 7 days.

Stage 3 of the Sarnat grading system is characterized by stupor with response to only strong stimuli, with withdrawal or decerebrate posturing, severe hypotonia (e.g. flaccidity) and suppression of deep tendon reflexes, primitive (e.g. Moro, tonic neck, suck) reflexes, and brainstem (e.g. corneal, oculocephalic, gag) reflexes. Clinical seizures are less frequent in stage 3 than in stage 2. The patient has generalized sympathetic or parasympathetic autonomic dysfunction with abnormal respiration and small or midposition pupils that are poorly reactive to light. EEG shows a deepened periodic pattern with increased amplitude and decreased frequency of bursts (every 6-12 seconds). Further worsening of the picture leads to a very-low-voltage or isoelectric EEG.

As used herein, a "child" brain injury, especially a child traumatic brain injury encompasses, or consists of, a brain injury, especially a traumatic brain injury, that is caused to a human individual ranging from more than one year old to twelve years old.

As used herein, an "adult" brain injury, especially an adult traumatic brain injury, encompasses, or consists of, a brain injury, especially a traumatic brain injury, that is caused to a human individual being from more than twelve years old to the age of death.

As used herein, a "derivative" of a HIP/PAP protein encompasses polypeptides comprising an amino acid sequence having at least 90% amino acid identity with a polypeptide selected from the group consisting of SEQ ID N° 1 to 3.

Comprises/comprising and grammatical variations thereof when used in this specification are to be taken to specify the presence of the stated features, integers, steps or components or groups thereof, but do not preclude the presence or addition of one or more other features, integers, steps, components or groups thereof.

A HIP/PAP protein derivative encompasses proteins comprising an amino acid sequence of at least 90 %, and preferably of at least 91%, 92%, 93%, 94%, 95%, 96%, 97%, 98%, 99%, or more amino acid identity with a polypeptide selected from the group consisting of the polypeptides of SEQ ID N° 1 to 3.

In some embodiments, a HIP/PAP protein derivative encompasses a protein having a strong amino acid identity with a HIP/PAP protein selected from the group consisting of SEQ ID N° 1-3, and possessing, as compared with the HIP/PAP protein of reference, one or more additions, substitutions or deletions of an amino acid residue. According to these embodiments, the said HIP/PAP protein derivative may comprise 1, 2, 3, 4, 5, 6, 7, 8, 9, 10, 11, 12, 13, 14, 15, 16, 17, 18, 19, 20 or more additions, substitutions or deletions of an amino acid residue, as compared with the amino acid sequence of the HIP/PAP protein of reference. According to these embodiments, the said HIP/PAP protein may possess not more than 25 additions, substitutions or deletions of an amino acid residue, as compared with the amino acid sequence of the HIP/PAP protein of reference.

As intended herein, a HIP/PAP protein derivative is biologically active. As intended herein, a HIP/PAP protein derivative encompasses a biologically active portion of a HIP/PAP protein.

A "biologically active" derivative of a HIP/PAP protein includes a HIP/PAP-derived peptide that possesses one or more of the following biological activities :
- (i) preventing *in vitro* the N-methyl-D-aspartate (NMDA)-induced neuronal cell death in mice neuronal primary cultures, particularly in mice cortical neuron primary culture and in mice cerebellum granular cell primary culture- (ii) preventing *in vivo* the excitotoxic lesions caused by the glutamanergic analogue ibotenate in the developing brain, e.g. the mouse developing brain;
- (iii) possessing an *in vivo* rescuing effect on the excitotoxic lesions caused by the glutamanergic analogue ibotenate in the developing brain, e.g. the mouse developing brain;
- (iv) affecting neuroprotection against neonatal neuronal cell death induced by a reactive oxygen species like hydrogen peroxide (H₂O₂).;
- (v) preventing in vitro the H₂O₂-induced damages on primary mice cerebellum granular cells and
- (vi) inducing changes in the production, phosphorylation and distribution of Gap-43

A "biologically active" derivative of a HIP/PAP protein with a biological activity selected among (i)-(v) above, is not necessarily endowed with the same order of magnitude of the said biological activity(ies), provided that the said HIP/PAP protein derivative possesses one or more of the biological activities (i)-(iv) above at a detectable level, using the assays disclosed in the examples herein.

In some embodiments, a HIP/PAP protein or a derivative thereof, including a biologically active portion of HIP/PAP as described above, can be isolated from cell or tissue sources by an appropriate purification scheme using standard protein purification techniques.

In other embodiments, a HIP/PAP protein or a derivative thereof, including a biologically active portion of HIP/PAP as described above, may be produced by recombinant DNA techniques that are familiar to one skilled in the art.

According to further embodiments, a HIP/PAP protein or a derivative thereof, including a biologically active portion of HIP/PAP as described above, may be synthesised chemically using standard peptide synthesis techniques.

An isolated or purified HIP/PAP protein or derivative thereof, including a biologically active portion of HIP/PAP as described above, is substantially free of cellular material or other contamination proteins from the cell or tissue source from which the said protein is derived, or substantially free from chemical precursors when chemically synthesised.

HIP/PAP protein derivatives also encompass HIP/PAP chimeric or fusion proteins. As used herein, a chimeric protein or a fusion protein comprises the polypeptides cited above which are fused to a non-HIP/PAP polypeptide. Within the fusion protein, the HIP/PAP polypeptide and the non-HIP/PAP polypeptide are fused to each other. The non-HIP/PAP polypeptide can be fused to the N-terminus or to the C-terminus of the HIP/PAP polypeptide.

For example, in one embodiment, the fusion protein is a GST-HIP/PAP fusion protein in which the HIP/PAP sequence is fused to the C-terminus of the GST sequence. Such fusion proteins can facilitate the purification of recombinant HIP/PAP.

In all cases the fusion proteins of the invention are "biologically active" according to the previous definition of this expression herein, which includes that the said fusion proteins possess the same biological activity as the HIP/PAP of SEQ ID N°3.

To determine the percent identity of two amino acid sequences for the purposes of the instant invention, the sequences are aligned for optimal comparison purposes. For example, gaps can be introduced in one or both of a first and a second amino acid sequence for optimal alignment and non-homologous sequences can be disregarded for comparison purposes.

For optimal comparison purposes, the percent identity of two amino acid sequences can be achieved with CLUSTAL W (version 1.82) with the following parameters : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent» ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Biologically active portions of HIP/PAP include peptides comprising amino acid sequences sufficiently homologous to the full length amino acid sequence of HIP/PAP of any one of SEQ ID N°1 to 3, which include the same number of amino acids than the corresponding full length HIP/PAP, and exhibit at least the same biological activity than HIP/PAP.

Biologically active portions of HIP/PAP include further peptides comprising amino acid sequences sufficiently homologous to the full length amino acid sequence of HIP/PAP of any one of SEQ ID N°1 to 3, which include more amino acids than the corresponding full length HIP/PAP, and exhibit at least the same biological activity than HIP/PAP.

In addition to naturally occurring allelic variants of the biologically active portion of HIP/PAP sequences that exist in mammals, the person skilled in the art will further appreciate that changes can be introduced by mutation into the nucleotide sequence of any one of SEQ ID N°1 to 3, thereby leading to changes in the amino acid sequence of HIP/PAP without altering the biological activity of HIP/PAP.

In addition, substitutions of non-essential amino acid can be made in the sequences corresponding to a HIP/PAP protein. A "non essential" amino acid residue is an amino acid residue that can be altered from the wild type sequence of HIP/PAP without altering the biological activity, whereas an "essential" amino acid residue is required for biological activity.

Generally, HIP/PAP protein derivatives encompass substances comprising a HIP/PAP protein or a HIP/PAP biologically active portion thereof.

In certain embodiments of these HIP/PAP protein derivatives, the HIP/PAP protein of the HIP/PAP biologically active portion thereof may be combined or associated with the non-HIP/PAP portion thereof through non-covalent bonds. Illustrative embodiments encompass liposomes comprising a HIP/PAP protein or a HIP/PAP biologically active portion thereof, wherein the non-HIP/PAP portion thereof consist of the liposome particles themselves. Depending of the kind of liposomes that are considered, or alternatively depending on the process used for manufacturing the said liposomes, the HIP/PAP protein, or the biologically active portion thereof, may be located at the liposome surface (e.g. exposed to the liposome outer environment) or alternatively encapsulated therein.

In certain other embodiments of these HIP/PAP protein derivatives, the HIP/PAP protein of the HIP/PAP biologically active portion thereof may be combined or associated with the non-HIP/PAP portion thereof through non-covalent bonds. Illustratively, the HIP/PAP PAP protein, or the biologically active portion thereof, may be covalently bound to a non-HIP/PAP portion selected form a protein or to a non-protein compound, like a polyethylene glycol molecule, wherein the final HIP/PAP derivative consists of a pegylated HIP/PAP or of a pegylated biologically active portion thereof.

In a HIP/PAP protein derivative that is considered in its state prior to its administration to a patient in need thereof, the said protein derivative may not be "biologically active", provided that the said HIP/PAP protein is biologically active once administered to the said patient.

Also, in a HIP/PAP protein derivative that is considered in its state prior to its administration to a patient in need thereof, the said protein derivative may already be "biologically active".

Generally, when used for preventing or treating a brain injury, or for any other HIP/PAP uses disclosed herein, a HIP/PAP protein or a protein derivative thereof is comprised in a pharmaceutical composition at an effective amount, in combination with one or more physiologically acceptable excipients.

Without wishing to be bound to any particular theory, the inventors believe that the complete HIP/PAP protein of sequence SEQ ID N°2, i.e. a polypeptide comprising the CRD sequence, and a pro-peptide, leads to a best folding of said protein, particularly when said protein is produced through DNA recombinant techniques in eukaryotic cells that have been transfected with an expression cassette encoding it. Incidentally, a correct folding of the therapeutically active HIP/PAP may improve biological efficiency for the pharmaceutical composition comprising said protein, for preventing or treating a brain injury caused by hypoxia, as compared to a composition comprising only a portion of the protein.

However, other forms of a HIP/PAP protein including the polypeptide forms of SEQ ID N°1 and 2, as well as any one of the HIP/PAP derivatives described in the present specification, may be used *in vitro* and *in vivo,* even if these polypeptides do not necessarily consist of the most preferred embodiments of the instant invention.

In a more general aspect, the present disclosure relates to the use of a HIP/PAP protein in a method for treating neonatal brain injury caused by a brain hypoxia. Without wishing to be bound by
any particular theory, the inventors believe that the various effects of the HIP/PAP protein on the *in vitro* and *in vivo* neonatal brain injury that are disclosed herein may also be useful in very specific patho-physiological situations of the adult or child brain selected from the group consisting of cerebrovascular accidents.

Thus, the said adult or child brain injury consists of a cerebrovascular accident, which may be also termed stroke.

A cerebrovascular accident or stroke consists of a rapidly loss of brain functions due to a disturbance in the blood vessels supplying blood to the brain. A cerebrovascular accident encompasses (i) an ischemia caused by thrombosis or embolism, which may be also termed "ischemic stroke" and (ii) an ischemia due to a haemorrhage, which may be also termed "hemorrhagic stroke". Like neonatal brain injury, a cerebrovascular accident or stroke may cause permanent neurological damage and may lead to death. According to the World Health Organisation, a cerebroventricular accident or stroke consists of a neurological deficit of cerebrovascular cause that persists beyond 24 hours or is interrupted by death within 24 hours.

A cerebrovascular accident or stroke encompasses thrombotic stroke, embolic stroke, ischemia caused by a cardiac dysfunction, systemic hypoperfusion, venous thrombosis and intracerebral haemorrhage.

Traumatic brain injuries generally cause an impairment of the brain oxygen flow resulting from cerebral hemorrhages, hematomas or edema.

In some embodiments, the adult or child brain traumatic injury belongs to brain injuries caused by hypoxia.

Traumatic brain injury (TBI) occurs when physical trauma injures the brain and is generally classified as mild, moderate, or severe, depending on the extent of loss of consciousness, loss of memory, and score on a neurological scale following the injury. Diagnosis and scaling of traumatic brain injury is conventionally performed by a method selected from the group consisting of Glascow Coma Scale (GCS) (Arlinghaus et al., 2005, Textbook of Traumatic Brain Injury, Washington, DC : American Psychiatric Association, pp; 59-62), a method of determination of the duration of post-traumatic amnesia (PTA), and a method of scaling the loss of consciousness (LOC) (Rao et al., 2000, Psychosomatics, Vol. 41 (n°2) : 95-103). It is generally agreed that a TBI with a GCS of 13 or above is mild, whereas a GCS of 9 to 12 is moderate and a GCS of 8 or below is severe. Illustratively, a GCS score of 13 to 15 is determined for a person whom duration of post-traumatic amnesia (PTA) is of less than 24 hours and whom duration of the loss of consciousness (LOC) ranges between 0 and 30 minutes. Yet illustratively, a GCS score of 9 to 12 is determined for a person whom duration of post-traumatic amnesia (PTA) ranges between 1 and 7 days and whom duration of the loss of consciousness (LOC) ranges between 30 minutes and 24 hours. Still illustratively, a GCS score of 3 to 8 is determined for a person whom duration of post-traumatic amnesia (PTA) is of more than 7 days and whom duration of the loss of consciousness (LOC) is of more than 24 hours.

Among the physiological similarities between Trauma brain injury and neonatal brain injury, TBI is accompanied by haemorrhages due to rupture of blood vessels which causes hypoxia.

HIP/PAP protein or a protein derivative thereof may be used for preventing or treating traumatic brain injuries having GCS classification scores ranging form 3 to 15. Thus, HIP/PAP protein or a protein derivative thereof may be used for preventing or treating traumatic brain injuries selected from the group consisting of "mild" TBI (GSC score ranging from 13 to 15), moderate TBI (GCS score ranging from 9 to 12) and severe TBI (GSC score ranging from 3 to 8). Typically, mild TBI have a GCS score ranging from 13 to 15, a PTA score less than one hour and a LOC score less than 30 minutes. Typically, moderate TBI have a GCS score ranging from 9 to 12, a PTA score ranging from 30 minutes to 24 hours and a LOC score ranging from 1 to 24 hours. Typically, severe TBI have a GCS score ranging from 3 to 8, a PTA score of more than one day and a LOC score of more than one day.

It is believed that a HIP/PAP protein will prove to be therapeutically useful in methods for treating TBI in general, irrespective of the GSC score that has been determined for the patient to be treated.

The therapeutic usefulness of HIP/PAP for treating TBI encompasses treatment of direct traumatic cerebellar injury is much less common than frontal or temporal TBIs. However, the cerebellum is often affected even when the initial injury does not directly involve this structure. Such indirect injury may induce a plurality of neurological disorders including, postural instability, tremor, impairments in balance and fine motor skills and cognitive deficits. Even if the causes of indirect cerebellum damage resulting from supratentorial trauma are not completely understood, several studies suggest the involvement of excitotoxicity (excessive release of the neurotransmitter glutamate) in the transmission of injury to cerebellum (Potts et al., Cerebellum, 2009, (8), 211-221).

Without wishing to be bound by any particular theory, the inventors believe that a HIP/PAP protein or a derivative thereof can protect cerebellum neurons from the indirect cerebellum damage resulting from TBI in child or adult patients by protecting cerebellum cell from excitotoxicity.

Thus, in some embodiments, the present specification also relates to the use of a HIP/PAP protein or derivative thereof for treating or preventing indirect traumatic cerebellum injury resulting from TBI.

It is believed that the HIP/PAP protein shall be administered to the patients affected with TBI during the acute phase, although it may also be administered during the chronic phase.

TBI encompasses focal or diffuse TBI. Diffuse TBI includes oedema and diffuse axonal injury, which is widespread damage to axons including white matter tracts and projection to the cortex. Diffuse TBI also encompasses concussion and diffuse axonal injury, wide spread damage to axons in areas including white matter and the cerebral hemispheres.

For focal TBI, the most common areas to have focal lesions in on-penetrating TBI are the orbitofrontal cortex and the anterior temporal lobes, which consist of areas that are involved in social behavior, emotion regulation, olfaction and decision-making.. One type of focal injury, cerebral laceration, occurs when the brain tissue is cut or torn.

Traumatic brain injuries include, without being limited to, frontal TBI, temporal TBI, direct cerebellar TBI and indirect cerebellar TBI.

As already mentioned in the present specification, a HIP/PAP protein or derivative thereof can protect *in vitro* cerebellar neurons from excitotoxic and oxidative death.

Without wishing to be bound by any particular theory, the inventors believe that a HIP/PAP protein or a derivative thereof can protect cerebellar neurons from death which occurs in various cerebellar disorders such as cerebellar ataxias.

In such conditions, a cerebellar atrophy may be observed.

Thus, the present specification also relates to the use of a HIP/PAP protein or a derivative thereof for treating or preventing a cerebellar ataxia.

As used herein, cerebellar ataxia refers to dysfunction of the cerebellum. Cerebellar ataxias may cause a variety of elementary neurological deficits, such as antagonist hypotonia, asynergy, dysmetria, dyschronometria, and dysdiadochokinesia.

Cerebellar ataxias encompass inherited spinocerebellar ataxias and acquired cerebellar ataxias.

In some embodiment, the present specification relates to the use of a HIP/PAP protein or a derivative thereof for treating inherent spinocerebellar ataxias.

Inherent spinocerebellar ataxias, include, without being limited to, autosomal dominant spinocerebellar ataxias (SCAs) and autosomal recessive spinocerebellar ataxias.

Examples of autosomal dominant spinocerebellar ataxias include SCA1 (autosomal dominant type 1), SCA2, SCA3, SCA4, SCA6, SCA7, SCA8, SCA9, SCA10, SCA11, SCA12, SCA13, episodic ataxia type 1 (EA-1) and episodic ataxia type 2 (EA-2).

Autosomal recessive spinocerebellar ataxias include, without being limited to, Friedreich's ataxia, ataxia telangiectasia and infantile-onset spinocerebellar ataxia.

In some embodiments, the present specification relates to the use of a HIP/PAP protein or a derivative thereof for treating and preventing acquired cerebellar ataxias.

Acquired cerebellar ataxias include, without being limited to, paraneoplastic syndrome, intoxication-induced cerebellar ataxias, infectious ataxias and vascular cerebellar ataxias.

Intoxication-induced cerebellar ataxia may be caused by exposure to toxics such as solvents, gasoline and heavy metals, ingestion of products such as alcohol, barbiturates or lithium and cytotoxic chemotherapeutic drugs.

Infectious ataxias encompass ataxias caused by viral, bacterial or prion infections.

For example, viral infectious cerebellar ataxias may result from viral and post-viral encephalopathies.

Bacterial infectious cerebellar ataxias include, without being to, ataxias resulting from meningovascular syphilis or Lyme disease.

Vascular ataxias include, without being limited to, ataxias caused by ischemic cerebrovascular accident including cerebellar infarction, hemorrhage or subdural hematoma.

As used herein, "brain injury", irrespective of whether the said brain injury consists of a neonatal brain injury, a child brain injury or an adult brain injury, does not encompass brain injuries that are initially caused by a demyelination of neuronal cells. In other words, "brain injury" does not encompass injuries caused to the brain by a demyelinating disease, irrespective of the kind of the demyelinating disease, e.g. auto-immune or inherited disease. Thus, as intended herein, "brain injury" does not encompass a brain injury caused by a disease selected from the group consisting of multiple sclerosis, acute disseminated encephalomyelitis, transverse myelitis, chronic inflammatory demyelinating polyneuropathy, Guillain-Barré Syndrome, central pontine myelinosis, Tay-Sachs disease, as well as inherited demyelinating diseases such as leucodistrophy diseases and Charcot Marie Tooth. As used herein, leucodistrophy diseases encompass adrenoleucodistrophy (WHO Classification #E71.3), adrenomyeloneuropathy (WHO Classification #E71.3), metachromatic leukodistrophy (WHO Classification #E75.2), Krabbe disease (WHO Classification #E75.2), Pelizaeus-Merzbacher disease (WHO Classification #E75.2), Canavan disease, childhood ataxia with central hypomyélination (CACH), leuloencephalopathy with vanishing white matter, Alexander disease, Refsum disease (WHO Classification #G60.1), Zellweger disease, Aicardi-Goutieres syndrom, megaloencephalic leukodistrophy and cerebrotendineous xanthomatosis.

As already mentioned in the present specification, because of the HIP/PAP activity on the Gap-43 production or phosphorylation that has been shown herein, a HIP/PAP protein or a derivative thereof may also be used *in vitro* and *in vivo* for modulating, enhancing or improving neuronal growth or plasticity mediated by Gap-43, a neuronal growth-related protein.

This invention thus also concerns the use of the HIP/PAP protein or a protein derivative thereof for preventing or treating a disorder or a disease involving a defect or a deregulation of Gap-43 production or a defect or a deregulation of Gap-43 phosphorylation, which includes Alzheimer's disease.

It is worth mentioning that HIP/PAP-related proteins have already been alleged in the art to be useful for acting on Alzheimer's disease, but without any starting experimental result that would technically support such activity allegations.

In contrast, the biological activity of a HIP/PAP protein on the production or on the phosphorylation of the Gap-43 protein supports for the fist time the usefulness of the said HIP/PAP protein for preventing or treating Alzheimer's disease.

Indeed, it is well known from the one skilled in the art that a defect in the production or in the phosphorylation of the Gap-43 protein occurs in Alzheimer's disease. Notably, it has been shown in the art that the production of an inactive Gap-43 protein is involved in patients affected with an Alzheimer's disease. Also, an altered phosphorylation of Gap-43 has previously been shown in the art in patients affected with an Alzheimer's disease.

Thus, this specification also relates to the use of a HIP/PAP protein or a derivative thereof for preventing or treating an Alzheimer's disease.

The present specification also concerns a method for preventing or treating an Alzheimer's disease comprising a step of administering, to a patient in need thereof, a HIP/PAP protein or a derivative thereof.

This specification also relates to the use of the HIP/PAP protein or a protein derivative thereof for preventing or treating pathological states that precede the occurrence of the Alzheimer's disease *per se,* which includes mind cognitive impairment.

In other words, this specification also pertains to the use of the HIP/PAP protein or a protein derivative thereof for preventing or treating mind cognitive impairments that occur prior to the Alzheimer's disease *per se.*

Because it has been shown in the examples herein that a HIP/PAP protein has the ability to rescue neuronal cells *in vitro,* then a HIP/PAP protein or a derivative thereof has also been proved to be useful as an agent for maintaining or improving the viability or the growth of cultured neuronal cells. Then, the results of the examples herein support the usefulness of a HIP/PAP protein or of a derivative thereof as a neuronal cell culture agent that may be added in a neuronal cell culture medium.

This specification also encompasses the use of a HIP/PAP protein for the *in vitro* culturing of neuronal cells, including for primary cultures of neuronal cells and for cultures of neuronal cell lines.

The present specification also pertains to a culture medium for neuronal cells comprising a HIP/PAP protein or a derivative thereof. Apart the presence of a HIP/PAP protein or of a derivative thereof, the qualitative and quantitative constitution of an appropriate culture medium is easily determined by the one skilled in the art. Illustratively, the HIP/PAP protein or the derivative thereof may simply be added to a known culture medium suitable for neuronal cells, e.g. in a RPMI 199 culture medium.

The said culture medium comprises an effective amount of the said HIP/PAP protein or of the said derivative thereof. The final concentration of a HIP/PAP protein or a derivative thereof in a culture medium above preferably ranges from 1 ng/mL to 10 mg/ml.

Regarding the *in vivo* uses, the HIP/PAP protein or a protein derivative thereof is preferably comprised as an active ingredient in a pharmaceutical composition.

The said pharmaceutical composition comprises the HIP/PAP protein, or a protein derivative thereof, and one or more pharmaceutically acceptable excipients.

The said pharmaceutical composition comprises en effective amount of the HIP/PAP protein, or a protein derivative thereof.

Usually, a pharmaceutical composition that may be used according to the invention comprises from 0.1 % by weight to 99.9% by weight of the HIP/PAP protein, or a protein derivative thereof, based on the total weight of the pharmaceutical composition. Thus, a pharmaceutical composition according to the invention may comprise at least 0.5%, 1%, 2%, 3%, 4%, 5%, 6%, 7%, 8%, 9%, 10%, 20%, 30%, 40%, 50%, 60%, 70%, 80%, 90% by weight or more of the HIP/PAP protein, or a protein derivative thereof, based on the total weight of the pharmaceutical composition. Also, a pharmaceutical composition according to the invention may comprise up to 10% 20%, 30%, 40%, 50%, 60%, 70%, 80%, 90%, 91%, 92%, 93%, 94%, 95%, 96%, 97%, 98%, 99%, 99.5% by weight or less of one or more pharmaceutically acceptable excipients, based on the total weight of the pharmaceutical composition.

The therapeutically effective dose of the HIP/PAP protein or a protein derivative thereof will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. The clinician will administer the HIP/PAP protein or a protein derivative thereof until a dosage is reached that achieves the desired effect for treatment of the condition in question.

The condition to be treated refers to one of the pathological conditions which are described in the present application such as a neonatal brain injury caused by hypoxia, an adult or child brain injury caused by hypoxia such as cerebrovascular accident, TBIs and cerebellar disorders.

As used herein, the term "patient" refers to a neonate patient, a child patient, or an adult patient, the latter including a pregnant patient.

As used herein more particularly, an effective amount of the HIP/PAP protein or of the protein derivative thereof consists of the amount of HIP/PAP protein or derivative thereof that can at least partially reverse the physical damages caused to the brain during a neonatal brain injury. Particularly, an effective amount of the HPI/PAP protein or of the protein derivative thereof consists of the amount of HIP/PAP protein or derivative thereof that reduces or blocks neuronal cell death occurring during neonatal brain injury.

Starting form the results shown in the examples herein, the effective amount of the HIP/PAP protein or of the protein derivative thereof may range from about 0.1 µg/kg of body weight and to about 100 mg/kg of body weight. Thus, according to the invention, an effective amount of the HIP/PAP protein or of the protein derivative thereof encompasses amounts of at least 1 µg/kg, 2 µg/kg, 3 µg/kg, 4 µg/kg, 5 µg/kg, 6 µg/kg, 7 µg/kg, 8 µg/kg, 9 µg/kg, 10 µg/kg, 15 µg/kg, 20 µg/kg, 20, µg/kg, 30 µg/kg, 40 µg/kg, 50 µg/kg, 60 µg/kg, 70 µg/kg, 80 µg/kg, 90 µg/kg, 100 µg/kg, 200 µg/kg, 300 µg/kg, 400 µg/kg, 500 µg/kg, 600 µg/kg, 700 µg/kg, 800 µg/kg, 900 µg/kg, 1 mg/kg or more of body weight of the patient.

In the case of neonatal injury, the term "patient" refers to the neonate patient including foetus as well as the pregnant patient.

To prevent or treat a neonatal brain injury to the foetus body, a pharmaceutical composition according to the invention is preferably administered to the pregnant adult body. However, in some embodiments, the said pharmaceutical composition may be administered directly to the foetus body, using specific endoscopy medical instrumentation.

To prevent or treat a neonatal brain injury of the neonate during delivery or subsequent to delivery, as well as to prevent or treat a brain injury of the adult, a pharmaceutical composition according to the invention is administered to the neonate body or to the adult body, respectively.

In certain embodiments, a pharmaceutical composition according to the invention is administered systemically.

In certain other embodiments, a pharmaceutical composition according to the invention is administered locally via an intracranial route, especially to the foetus body or the neonate body where skull bone formation is not terminated.

The route of the HIP/PAP protein or of the protein derivative thereof administration is in accord with known methods, e.g., by injection or infusion by intravenous, intramuscular, intracerebral, intraperitoneal, intracerobrospinal, subcutaneous, oral, topical, or inhalation routes, or by sustained-release systems as noted below.

In some embodiments, the HIP/PAP protein or of the protein derivative thereof, or alternatively the pharmaceutical composition containing the same, is administered as a single dose.

In some other embodiments, the HIP/PAP protein or of the protein derivative thereof, or alternatively the pharmaceutical composition containing the same, is administered as a plurality of doses at determined time intervals, e.g. starting from the time of brain injury diagnosis or from the time at which brain injury expectation is diagnosed, and until the time upon which the treated body is out of danger.

In certain embodiments of a pharmaceutical composition according to the invention, the HIP/PAP protein or of the protein derivative thereof, may be combined with one or more other active ingredients useful for preventing or treating a brain injury caused by a brain hypoxia, including one or more active ingredients already known in the art for being useful for preventing or treating a neonatal brain injury.

Therapeutic compositions according to the invention may be prepared for storage by mixing the desired molecule having the appropriate degree of purity with optional pharmaceutically acceptable carriers, excipients, or stabilizers (Remington's Pharmaceutical Sciences, 16th edition, Osol, A. ed. (1980)), in the form of lyophilized formulations or aqueous solutions. Acceptable carriers, excipients, or stabilizers are non toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatine, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN.TM., PLURONICS.TM. or polyethylene glycol (PEG).

Additional examples of such carriers include ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms of the HIP/PAP or derivative thereof substances according to the invention include polysaccharides such as sodium carboxymethylcellulose or methylcellulose, polyvinylpyrrolidone, polyacrylates, polyoxyethylene-polyoxypropylene-blo- ck polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The HIP/PAP protein or the protein derivative thereof will typically be formulated in such vehicles at a concentration of about 0.1 mg/ml to 100 mg/ml.

The HPI/PAP protein or the protein derivative thereof to be used for *in vivo* administration must be sterile. This is readily accomplished by filtration through sterile filtration membranes, prior to or following lyophilization and reconstitution. The HIP/PAP protein or of the protein derivative thereof will ordinarily be stored in lyophilized form or in solution if administered systemically. If in lyophilized form, the HPI/PAP protein or the protein derivative thereof is typically formulated in combination with other ingredients for reconstitution with an appropriate diluent at the moment of use. An example of a liquid formulation of the HIP/PAP protein or the protein derivative thereof is a sterile, clear, colorless unpreserved solution filled in a single-dose vial for subcutaneous injection. Preserved pharmaceutical compositions suitable for repeated use may contain, for example, depending mainly on the indication and type of polypeptide:
a) the HIP/PAP protein or the protein derivative thereof;
b) a buffer capable of maintaining the pH in a range of maximum stability of the polypeptide or other molecule in solution, preferably about 4-8;
c) a detergent/surfactant primarily to stabilize the polypeptide or molecule against agitation-induced aggregation;
d) an isotonifier;
e) a preservative selected from the group of phenol, benzyl alcohol and a benzethonium halide, e.g., chloride; and
f) water.

If the detergent employed is non-ionic, it may, for example, be polysorbates (e.g., Polysorbate®, Tween®.) 20,80, etc.) or poloxamers (e.g., Poloxamer®. 188). The use of non-ionic surfactants permits the formulation to be exposed to shear surface stresses without causing denaturation of the polypeptide. Furthermore, such surfactant-containing formulations may be employed in aerosol devices such as those used in a pulmonary dosing, and needleless jet injector guns (see, e.g., EP 257,956).

An isotonifier may be present to ensure isotonicity of a liquid composition of the HIP/PAP protein or the protein derivative thereof, and includes polyhydric sugar alcohols, preferably trihydric or higher sugar alcohols, such as glycerin, erythritol, arabitol, xylitol, sorbitol, and mannitol. These sugar alcohols can be used alone or in combination. Alternatively, sodium chloride or other appropriate inorganic salts may be used to render the solutions isotonic.

The buffer may, for example, be an acetate, citrate, succinate, or phosphate buffer depending on the pH desired. The pH of one type of liquid formulation of this invention is buffered in the range of about 4 to 8, preferably about physiological pH.

The preservatives phenol, benzyl alcohol and benzethonium halides, e.g., chloride, are known antimicrobial agents that may be employed.

Compositions of the therapeutic HIP/PAP protein or protein derivative thereof are generally placed into a container having a sterile access port, for example, an intravenous solution bag or vial having a stopper pierceable by a hypodermic injection needle. The formulations are preferably administered as repeated intravenous (i.v.), subcutaneous (s.c.), or intramuscular (i.m.) injections, or as aerosol formulations suitable for intranasal or intrapulmonary delivery (for intrapulmonary delivery see, e.g., EP 257,956).

The HIP/PAP protein or the protein derivative thereof can also be administered in the form of sustained-released preparations. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacr- ylate) as described by Langer et al., J. Biomed. Mater. Res., 15: 167-277 (1981) and Langer, Chem. Tech., 12: 98-105 (1982) orpoly(vinylalcohol)), polylactides (U.S. Pat. No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer et al., supra), degradable lactic acid-glycolic acid copolymers such as the Lupron Depot.TM. (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C., resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release HIP/PAP protein or protein derivative thereof also include liposomally entrapped polypeptides.. Liposomes containing the polypeptide of interest are prepared by methods known per se: DE 3,218,121; Epstein et al., Proc. Natl. Acad. Sci. USA, 82: 3688-3692 (1985); Hwang et al., Proc. Natl. Acad. Sci. USA, 77: 4030-4034 (1980); EP 52,322; EP 36,676; EP 88,046; EP 143,949; EP 142,641; Japanese patent application 83-118008; U.S. Pat. Nos. 4,485,045 and 4,544,545; and EP 102,324. Ordinarily the liposomes are of the small (about 200-800 Angstroms) unilamellar type in which the lipid content is greater than about 30 mol. % cholesterol, the selected proportion being adjusted for the optimal therapy.

The therapeutically effective dose of a HIP/PAP protein or protein derivative thereof will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. The clinician will administer the HIP/PAP protein or a protein derivative thereof until a dosage that achieves the desired effect for treatment of the condition in question is reached.

The route of administration is in accord with known methods, e.g. administration by injection or infusion by intravenous, intramuscular, intracerebral, intraperitoneal, intracerobrospinal, subcutaneous, intraocular, intraarticular, intrasynovial, intrathecal, oral, topical, or inhalation routes, or administration by sustained-release systems as noted below. The HIP/PAP or a protein derivative thereof also are suitably administered by intralesional or perilesional routes, to exert local as well as systemic therapeutic effects. Examples of pharmacologically acceptable salts of molecules that form salts and are useful hereunder include alkali metal salts (e.g., sodium salt, potassium salt), alkaline earth metal salts (e.g., calcium salt, magnesium salt), ammonium salts, organic base salts (e.g., pyridine salt, triethylamine salt), inorganic acid salts (e.g., hydrochloride, sulfate, nitrate), and salts of organic acid (e.g., acetate, oxalate, p-toluenesulfonate).

The HIP/PAP protein or the protein derivative thereof may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, supra.

Sustained-release preparations may be prepared. Suitable examples of sustained-release preparations include semipermeable matrices of solid hydrophobic polymers containing the HIP/PAP protein or a protein derivative thereof , which matrices are in the form of shaped articles, e.g. films or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C., resulting in a loss of biological activity y. Various rational strategies of stabilization can be devised, depending on the denaturation mechanism involved. These strategies may be achieved by modifying sulfhydryl residues, lyophilizing from acidic solutions, controlling moisture content, using appropriate additives, and developing specific polymer matrix compositions.

The instant invention is further illustrated by, without being limited by, the examples below.

### EXAMPLES

A recombinant human HIP/PAP protein was produced in an *E. coli* bacterial system, and then has been purified. The resulting product was called ALF-5755. Compared with HIP/PAP SEQ ID N° 2, ALF-5755 of SEQ ID N°3 has one single extra amino acid, a methionine, in the NH2 terminal end.

### Example 1 : In vitro effects of a HIP/PAP protein in a model of neonatal brain injury

### A. Materials and Methods

### Cortical Neurons Culture.

Primary cortical neurons cultures were prepared from E14.5 embryonic mice. Briefly, foetuses were removed from the uterus and placed in a sterile Petri dish. Brains were cut and placed in HBSS 1x with 1 M HEPES (Invitrogen). Using a dissecting microscope, brains, meninges, basal ganglia and hippocampus were dissected out and neocortex were put in Neurobasal medium containing B27 supplement (Invitrogen). Then, neocortex were dissociated with 0.25% Trypsin - 0.02% EDTA and incubated with DNase 1 (Sigma St-Quentin Fallavier, France). Cells were platting with MEM1X and 10% Horse serum at a density of 80000 cells/well in 96 wells plates coated with poly-DL-ornithine (Sigma St-Quentin Fallavier, France). Two to four hours after platting, the medium was removed and substituted with Neurobasal plus B27. Three days later, cells were treated with 5 µM of AraC (cytosine arabinoside) to curb glial proliferation. Between DIV10 and DIV12, neurons were treated for 8 hours with either one or the other product:
(i): PBS;
(ii): 3µg/ml or 5 µg/ml of ALF-5755;
(iii): 300 µM of NMDA;
(iv): 300 µM of NMDA plus 10 µM of MK801 or
(v): 300 µM of NMDA and 3µg/ml or 5 µg/ml of ALF-5755.

### Measurement of Cell viability

Cell viability was monitored using the MTS/Formazan assay (CellTiter 96 Aqueous One Solution Cell Proliferation Assay; Promega, Charbonnieres, France).

### Western blot analysis.

Total proteins were extracted from treated neuronal cultures (see below) and from the right neopallium of pups which has received at P5 intraparenchymal injections of either PBS, 10 µg of ibotenate, 0.3 µg of ALF-5755 (which has been determined as the best effective concentration of ALF-5755) or 10 µg ibotenate with 0.3 µg of ALF-5755. Pups were killed 4, 24, 48 or 120 hours after drugs administration. Proteins were extracted according to the protocol of a commercially available kit (Cell Signaling Technology).

Anti-Total-Gap43 and anti-Ser41-Phospho-Gap43 were used at a concentration of 1/1000. To standardize the protein expression among samples, we used an anti-b-tubulin goat antibody (Santa Cruz Biotechnology) at a concentration of 1/10000. Western blot experiments were run in duplicate. Differences in protein concentrations between samples were estimated using ImageJ.

### Immunocytochemistry.

For Total Gap-43, PhosphoGap43 and growth cone (2G13) detections, treated cell cultures were fixed in 4% PFA and reacted with chicken anti-Total-Gap43 antibody (Interchim; 1/200), rabbit anti-Ser41-PhosphoGap43 antibody (Novus Biologicals 1/200), mouse anti-Growth Cone 2G13 antibody (Novus Biologicals 1/200). Detection of labelled antigens was performed with a Cy3-conjugated donkey anti-chicken antibody, AMCA-conjugated donkey anti-rabbit antibody and Alexa 488-conjugated donkey anti-mouse antibody (Jackson Immunoresearch).

### Statistical Analysis

To study the effect of each treatment, the data were analyzed with a Kruskal-Wallis test.

### B. Results

A recombinant human HIP/PAP protein was produced in an *E. coli* bacterial system, and then has been purified. The resulting product was called ALF-5755. Compared with HIP/PAP SEQ ID N° 2, ALF-5755 of SEQ ID N°3has one single extra amino acid, a methionine, in the NH2 terminal end.

Biochemical, biological, and functional studies of ALF-5755 have been performed. They established that ALF-5755 exhibited the same biological activities, especially, mitogenic and antiapoptotic activities, as those previously demonstrated to pertain to HIP/PAP in several different animal models.

The neuroprotective efficacy of ALF-5755, *in vitro* against N-methyl-D-aspartate (NMDA)-induced cell death has been studied in primary mice cortical neuronal cultures.

The ALF-5755 *in vitro* neuroprotection properties have been evaluated, using a well-established model of primary neuronal cultures (neurons from cerebral hemisphere of E14
Swiss mice exposed to NMDA). The experiments were performed between day 8 and day 12 from the onset of the culture. Doses of HIP/PAP (ranging from 150 ng/ml to 10 µg/ml) were added to the culture medium immediately after or 3 hours after the beginning of an 8-hour intoxication by 300 µM of NMDA or by 10, 30, 100 µM of H₂O₂. Cell viability was then measured using a MTT assay.

The neuronal death after exposure to NMDA alone (without treatment with ALF-5755) was of 60% of the neurons. Treatment with ALF-5755 caused a significant, dose-dependent decrease of the proportion of neuronal death. The decrease in neuronal death was maximum (35% of neuronal death against 45% without ALF-5755) at 5 µg/mL of ALF-5755 when added immediately after the beginning of the insult. A neuroprotective effect of ALF-5755 was also observed when it was added 3 hours after the beginning of the insult.

Exposure to H₂O₂ alone induces a dose-dependent diminution of cell survival by oxidative damage, which is maximal at 100µM exposure (64% of neuronal death). Treatment with ALF-5755 totally or partially reverses the toxicity of H₂O₂. This protective effect is maximal for 3µg/ml of ALF-5755. Protective effects of ALF-5755 were comparable to those observed with catalase. Protective effects of ALF-5755 are observed whether the drug is added immediately after H₂0₂ or 3 hours after the insult. ALF-5755 shows anti-oxidant properties. Immunocytochemistry (ICC) studies and western blot (WB) analyses were performed on neuronal cultures to determine the effects of ALF-5755 on the production, phosphorylation status at serine 41 (S41) and distribution of GAP-43, a protein involved in axonal growth and neuronal plasticity.

When added immediately after NMDA, ICC demonstrates that ALF-5755 induces a fascicular distribution of GAP-43-labelled neuronal processes. This effect is optimal at 5µg/ml and is not observed in control cultures, in cultures treated with NMDA or H₂0₂, or in cultures treated with H₂0₂ and ALF-5755. Furthermore, ICC shows that ALF-5755 induces an increased expression of phospho-GAP-43 in neuronal processes, when compared to control cultures or to cultures treated with NMDA or H₂O₂ alone.

Western blot analyses of extracts from cultured neurons allowed to quantify changes of total GAP-43 and phospho-GAP-43 levels. NMDA did not induce any significant changes in the amount of GAP-43. In contrast, ALF-5755 alone induced a dose-dependent decrease of total GAP-43 but a significant increase of the proportion of phosphorylated GAP-43 (phospho GAP-43 / total Gap-43). The effect of ALF-5755 on GAP-43 was still more pronounced in cultures exposed to NMDA. These changes of in the phosphorylation status of GAP-43 induced by ALF-5755 could explain the fasciculation observed in cultures exposed to ALF-5755.

Altogether these data support the hypothesis that ALF-5755 prevents excitotoxic and oxidative neuronal cell death *in vitro.* Morever, ALF-5755 induces plasticity-like changes including GAP-43 phosphorylation and fasciculation.

### Example 2 : In vivo effects of a HIP/PAP protein in a model of neonatal brain injury

### A. Materials and Methods

### Animals

Swiss mice of both sexes were used in this study. Experimental protocols were approved by the institutional review committee and meet the guidelines of the 'Institut National de la Santé et de la Recherche Médicale'.

### Drugs

Ibotenate (Sigma, St-Quentin Fallavier, France), ALF-5755, NMDA (Tocris, Bristol, UK) and MK801 (Sigma, St-Quentin Fallavier, France) were diluted in phosphate-buffered saline (PBS).

### Administration of Excitotoxic Drugs

Excitotoxic brain lesions were induced by injecting ibotenate into developing mouse brains, as previously described (Marret et al., 1995; Gressens et al., 1997, 1998; Bac et al., 1998; Dommergues et al., 2000; Tahraoui et al., 2001; Laudenbach et al., 2001, 2002; Husson et al., 2002). Briefly, mouse pups were injected intracerebrally (into the neopallial parenchyma) on day P5. Intraparenchymal injections were performed with a 25-gauge needle on a 50 µl Hamilton syringe mounted on a calibrated microdispenser. The needle was inserted 2 mm under the external surface of scalp skin in the frontoparietal area of the right hemisphere, 2 mm from the midline in the lateral-medial plane, and 3 mm anterior to bregma in the rostro-caudal plane. Two 1 µl doses of ibotenate, or ibotenate plus ALF-5755 or PBS were injected at 20 s intervals. The first bolus corresponds to an injection localized in the white matter and the second bolus represents the cortical injection. 10 µg ibotenate or 10 µg ibotenate plus ALF-5755 (range: 0.0015 µg-0.15 µg) or PBS was administered to each pup.

For some pups, ALF-5755 has been injected intraperitonealy immediately or 3 hours after ibotenate injection at different concentrations (from 0.0015 µg to 1.5 µg).

### Experimental Groups

Pups from at least two different litters were used in each experimental group, and data were obtained from two or more successive experiments.

Three different groups were constituted depending on how (intraparachymal co-administered with ibotenate, intraperitoneal injected immediately or 1 to 5 hours after ibotenate injection) ALF-5755 was injected.

The first P5 pups group received an intraparenchymal injection of ibotenate with PBS (vehicle control) or with 0.003, 0.015, 0.03 or 0.3 µg of ALF-5755.

In the second set of experiments designed to compare the intraparenchymal injection with the intraperitoneal injection of ALF-5755, P5 pups received an intraparachymal injection of ibotenate with PBS and then an intraperitoneal injection of ALF-5755 at several concentrations (0.0015, 0.0075, 0.015, 0.075, 0.15, 0.75 or 1.5 µg of ALF-5755).

To know if ALF-5755 can repair, instead of preventing, ibotenate lesions, the third P5 mouse pups group received a delayed intraperitoneal injection of 0.75 or 1.5 µg of ALF-5755 3 hours after intraparenchymal injection of ibotenate. One or five hours after ibotenate injection, a single ALF-5755 concentration was tested intraperitonealy (1.5 µg).

### Determination of Lesion Size

12 mouse pups were used in each experimental group. Mouse pups were sacrificed by decapitation 120 h following the excitotoxic challenge. Brains were immediately fixed in 4% formalin for at least 4 days. Following embedding in paraffin, we cut 20 µm-thick coronal sections. Every third section was stained with cresyl-violet. Previous studies (Marret et al., 1995; Gressens et al., 1997; Husson et al., 2002) have shown an excellent correlation between the maximal size of the lesion in the lateral-medial and fronto-occipital axes of the excitotoxic lesions. This permitted an accurate and reproducible determination of the maximal fronto-occipital diameters of the lesions, which equals the number of sections where the lesion was present multiplied by 20 µm.

### B. Results

The neuroprotective potential of ALF-5755 *in vivo* was evaluated by means of a mouse model of neonatal excitotoxic brain lesions mimicking brain damage associated with cerebral palsy.

Brain damage was induced by ibotenate, a glutamanergic agonist acting on NMDA and metabotropic glutamate receptors. When injected into the murine neopallium at postnatal day (P) 5, ibotenate produced dramatic neuronal loss in neocortical layers and large periventricular white matter cysts resembling those seen in periventricular leukomalacia, which is a lesion appearing in numerous human premature newborns.

ALF-5755 was either co-injected with ibotenate intracerebrally at P5, or injected ALF-5755 intraperitoneally immediately, or 3 hours after ibotenate. The analysis of brain lesions was performed at P10.

Co-injection of ALF-5755 with ibotenate reduced white-matter and cortical gray-matter lesions by 37% and 67%, respectively, for a dose of 150 ng ALF-5755 (as compared with pups treated with ibotenate alone). A good (50% decrease of the size of the cerebral lesions) neuroprotection was also observed when the injection of ALF-5755 was performed intraperitoneally immediately, or 3 hours after insult. This establishes the efficacy of a single injection of ALF-5755, even delayed, via the systemic route. This also indicates that ALF5755 administered either intra-cerebrally or at the periphery can exhibit a therapeutic effect in neurological diseases and related diseases as defined above.

Further confirming the *in vitro* data, western blot analysis showed that ALF-5755, alone or in combination with ibotenate, induces a significant decrease in total GAP-43, but a significant and very important increase in the proportion of phosphorylated GAP-43 when compared to controls or animals injected with ibotenate alone.

### Example 3 : In vivo expression of a HIP/PAP protein in developing cerebellum of mouse pups and In vitro effects of ALF-5755 protein in a model of cerebellar injury

### A. Materials and Methods

### Quantitative RT PCR

RNAs from P0, P5-7, and P60 swiss mice cerebella, P5 cortices, and adults uterus were extracted using RNeasy mini kit (Quiagen). Reverse transcription was performed with 600ng of total RNAs to obtain HIP/PAP and HPRT cDNAs. Then quantitative PCR of HIP/PAP and HPRT transcripts (45 cycles of 30" denaturation at 94°C, 30" hybridisation at 58°C, and 30" polymerisation at 72°C with appropriate primers) was elicited with the Biorad system. Quantification of HIP/PAP expression in pups' cerebellum of various ages was obtained by rationalizing HIP/PAP against HPRT transcripts (associated Biorad software) to calibrate the RT PCR and compare levels of HIP/PAP expression among time.

### Cerebellar Granular Neurons Culture.

Granular cells represent the vast majority of cerebellar neurons. Primary granular neurons cultures were prepared from P7 mice pups. Briefly, brains were dissected and placed in HHGN (HBSS 1x with 1 M HEPES and 4.45% glucose; Invitrogen). Meninges were removed to avoid massive contamination by astrocytes. After isolation, the cerebella were washed in HHGN, reduced in small pieces and cells were dissociated with 0.25% Trypsin - 0.02% EDTA and incubated with DNase 1 (Sigma St-Quentin Fallavier, France). Cells were platted in MEM1X and 10% Horse serum at a density of 22 millions cells/well in 24 wells plates coated with poly-L-ornithine (Sigma).Twenty four hours post plating, 10 µM of AraC (cytosine arabinoside) was added to culture medium to curb glial proliferation. After 7 days *in vitro* (DIV7), neural death was induced by treating cells for 1 hour and allowing a subsequent recovering period of 8, 16 or 24 hours with the following inducers:
(i): H₂O₂ 0.01 to 1 mM to induce oxidative stress and achieve a subsequent cell death of 60%,
(ii): 0.3 to 3 mM NMDA to induce excito-toxicity and achieve a subsequent cell death of about 40%.

In both conditions, ALF-5755 neuro-protective effect was assessed at 5 and 10µg/mL in the presence of 0.3 mM of H₂O₂ concentration or in the presence of 1 mM of NMDA.

### Immunocytochemistry.

For GFAP (astrocyte detection), NeuN (neuron detection), Zic1 (mature granule cells detection), MAP2 (neuron detection), and clived caspase 3 (active caspase 3 detection) staining, treated cell cultures were fixed in 4% PFA and reacted with anti-GFAP (Dako, 1/500), anti-NeuN (Millipore, 1/100), anti-Zic1 (Abcam 1/100), anti-MAP2 (Abcam, 1/2000), , and anti-clived caspase 3 (Cell Signaling Technology, 1/100) antibodies. Detection of labelled antigens was performed with secondary antibodies fluorescing at various wave-lengths depending on the experiment, respectively Cy3-conjugated donkey anti-mouse antibody (1/1000, Jackson Immunoresearch), and 488-conjugated donkey anti-rabbit antibody (1/1000, Molecular Probes). Nuclei were then counter-stained with Dapi (1/1000).

### Statistical Analysis

Statistical analyses were performed with Graphpad software.

### B. Results

### HIP/PAP cerebellar expression during post-natal development:

So far, HIP/PAP expression in cortex and cerebellum of post-natal developing mice has never been documented. Here we report that HIP/PAP is expressed in both area with levels at P5 that are low however comparable. HIP/PAP level increases among time in cerebellum to reach a 20 folds magnitude in adults when compared to P5 mice. Expression in other structures is under investigation.

### Evaluation of ALF-5755 protective effect on H₂O₂ induced cell death:

One hour exposure to H₂O₂ at a concentration of 0.3mM and subsequent recovering time of 16H was shown to induce about 61% of cell death.

It was observed that cell H₂O₂ exposure did not elicit the clivage of caspase 3. Consequently, the H₂O₂-induced cell was independent of caspase 3 pathway
as.

Treatment with ALF-5755 at 5 or 10 µg/mL µM impacted neither on clived caspase 3 levels nor on AIF localization. However, cells exposed to ALF-5755 at 5 or 10 µg/ml display an improved morphology when compared to controls cells. The cells treated by ALF-5755 have a less advanced pyknotic aspect than that of control cells since the chromatin compaction is less important

Thus, this result shows that ALF5755 protein may preserve cerebellum granular cells from H₂O₂-induced cell death.

### Evaluation of ALF-5755 protective effect on NMDA induced cell death:

NMDA at a concentration of 1mM for 1 hour and a recovering time of 16 hours induced about 34% of cell death. It was observed that cell NMDA exposure elicited the clivage of caspase 3. Consequently, the NMDA-induced cell death was signaled by the activation of caspases pathway.
Treatment with ALF-5755 at 5 or 10 µM significantly reduced the occurrence of piknotic cells : in the control sample, about 80% of cells displayed piknotic aspect whereas in the presence ALF-5755 at 5 µM or at 10 µM, the percentage of piknotic cells did not exceed 40%.. Therefore, ALF-5755 was shown to protect granular cell neurons from NMDA induced cell death.

Taken together, these results strongly suggest that ALF-5755 may prevent cerebellum granular cells from both excitotoxic and oxidative death.

### Example 4 : Protective effects of a HIP/PAP protein against apoptosis of cortical neuronal cells.

### A. Materials and Methods

### A.1. Cortical culture

P0 pups were sacrificed by hypothermia; their cortex dissected aseptically in CMF-Tyrode solution. Meninges were removed, tissue were chopped into smaller pieces and collected in CMF-Tyrode. These were treated with trypsin-DNAse and then dissociated in the same solution by triturating to make a single cell suspension, pelleted and resuspended in Dulbecco's modified Eagle's medium-F-12 (DMEM-F-12) containing 15 mM HEPES, L-glutamine, pyroxidine hydrochloride (Invitrogen, Carlsbad, CA), N2 supplement (Invitrogen), 10% fetal calf serum, 25 mM KCI, and penicillin-streptomycin.

Cells were plated at a density of 2 × 10⁴ cells/cm² onto poly-D-Lysine -coated Labtek chamber (Nunc, Roskilde, DK). After 24 h of incubation at 37°C in 5% CO₂, the serum-containing medium was removed. The cells were then cultured in Serum free medium for 12 hours after which treatment with NMDA (final concentration of 0.3 mM) and HIP (final concentration of 18.3 ng) was started. The treatment continued for the next 24 hours after which cells were washed with 1X PBS and then fixed in 4% PFA.

### A.2. TUNEL assay

TUNEL kit was purchased from Roche and assay was performed according to manufacturer's suggestions. Briefly cells were permeabilized for 30 min in 0.02% Triton-X and incubated in labeling mixture consisting of terminal nucleotidyl transferase and labeled nucleotide mixture for 1 h at 37 C in a humidified chamber. Quantification of TUNEL-positive cells in cultures were performed by counting TUNEL positive cells from a total of 10 random fields from two wells each.

### B. Results

The results are summarized in Figure 1, where the level of cortical cell apoptosis is assessed by measuring the percent of TUNEL positive cells.

As it is shown in Figure 1, the HIP/PAP protein exerts a protective effect of the cortical neuronal cells, even when used alone (See the "HIP" bar at the extreme right part of Figure 1). These results mean that the HIP/PAP protein alone induces a protective effect against baseline effects of the *in vitro* cultivation of the cells.

Most importantly, Figure 1 shows that the HIP/PAP protein exerts a strong protective effect against the cortical neuronal cell apoptosis that is induced by NMDA (compare the "NMDA" bar with the "NMDA + HIP" bar.

The results shown in Figure 1 were obtained from *in vitro* cultures of cortical cells originating from mice that are homozygous for the wild-type GAP 43 gene (GAP43 +/+).

Although not shown, the same results were obtained by using *in vitro* cultures of cortical cells originating either (i) from GAP43 +/- mice or (ii) from GAP43 -/- mice.

The latter results suggest that the protective effect of the HIP/PAP protein which was seen did not require the presence of the GAP43 protein.

### Example 5 : Effects of a HIP/PAP protein on plasticity of neuronal cells

### A. Materials and Methods

### A.1.Cortical culture

P0 pups were sacrificed by hypothermia; their cortex dissected aseptically in CMF-Tyrode solution. Meninges were removed, tissue were chopped into smaller pieces and collected in CMF-Tyrode. These were treated with trypsin-DNAse and then dissociated in the same solution by triturating to make a single cell suspension, pelleted and resuspended in Dulbecco's modified Eagle's medium-F-12 (DMEM-F-12) containing 15 mM HEPES, L-glutamine, pyroxidine hydrochloride (Invitrogen, Carlsbad, CA), N2 supplement (Invitrogen), 10% fetal calf serum, 25 mM KCI, and penicillin-streptomycin.

Cells were plated at a density of 2 × 10⁴ cells/cm² onto poly-D-Lysine -coated Labtek chamber (Nunc, Roskilde, DK). After 24 h of incubation at 37°C in 5% CO₂, the serum-containing medium was removed. The cells were then cultured in Serum free medium for 12 hours after which treatment with NMDA (final concentration of 0.3 mM) and HIP (final concentration of 18.3 ng) was started. The treatment continued for the next 24 hours after which cells were washed with 1X PBS and then fixed in 4% PFA.

### A.2. Microscopy

Images were captured on a Zeiss Axioplan 2 microscope using an Axio Cam HRC CCD camera and analysis was done using the Zeiss KS 400 3.0 software. The images required minor adjustment of contrast and brightness. No additional image alteration was necessary.

### A.3. Neurite length analysis

The number of primary, secondary and tertiary neurites was looked at, besides looking at the number of branch points. Beta III tubulin was used as a marker (data not shown in the example). Images were captured on a Zeiss Axioplan II. The neurite length was obtained using IM50 software.

### B. Results

The results are depicted in Figures 2 to 6, respectively.

The results of Figures 2 to 5 show that the HIP/PAP protein, even when it is used alone, exerts an increase in the neuroplasticity, since the HIP/PAP protein (i) increases the number of primary neurites (Fig. 2), as well as the number of cells having secondary and tertiary neurites (See Figures 3 and 4, respectively).

The results depicted in Figure 6 show that the HIP/PAP protein has the ability to protect neuronal cells against the adverse effects caused by NMDA on the neurite growth. More precisely, the results presented in Figure 6 show that HIP/PAP, when added to neuronal cells incubated with NMDA, restores the level of neurite growth that is measured in control neuronal cell cultures incubated with the culture medium alone.

Further, it has been shown that the HIP/PAP protein has the ability to restore the neuroplasticity of cortical neuronal cells subjected to an incubation with a cytotoxic amount of NMDA.

The results shown in Figures 2, 5 and 6 were obtained from *in vitro* cultures of cortical cells originating from mice that are homozygous for the wild-type GAP 43 gene (GAP43 +/+).

Although not shown, the same results were obtained by using *in vitro* cultures of cortical cells originating either (i) from GAP43 +/- mice or (ii) from GAP43 -/- mice.

The latter results suggest that the positive effect of the HIP/PAP protein on neuroplasticity which was seen did not require the presence of the GAP43 protein.

## Claims

1. The "HIP/PAP" protein or a protein derivative thereof for use in preventing or treating a neonatal brain injury, which includes a neonatal brain injury caused by a brain hypoxia, wherein the "HIP/PAP" protein comprises an amino acid sequence having at least 90% amino acid identity with a polypeptide selected from the group consisting of the polypeptides of SEQ ID NO 1 to 3,
wherein the protein derivative thereof is selected from the group consisting of a biologically active portion of a "HIP/PAP" protein and "HIP/PAP" chimeric or fusion proteins,
and wherein the said neonatal brain injury encompasses a pathological state selected from the group consisting of foetal hypoxemia, perinatal hypoxemia, foetal ischemia, perinatal ischemia, and an excess release of excitatory neurotransmitters.

2. The "HIP/PAP" protein or protein derivative thereof for use according to claim 1, wherein the said neonatal brain injury consists of a cerebrovascular accident, which includes stroke.

3. The "HIP/PAP" protein or protein derivative thereof for use according to claim 1, wherein the said neonatal brain injury is selected from the group consisting of ischemic stroke and hemorrhagic stroke.

## Patentansprüche

1. "HIP/PAP"-Protein oder ein Proteinderivat davon zur Verwendung zur Vermeidung oder Behandlung einer neonatalen Hirnverletzung, die eine durch Hirnhypoxie verursachte neonatale Hirnverletzung enthält,
wobei das "HIP/PAP"-Protein eine Aminosäurensequenz mit mindestens 90% Aminosäurenidentität mit einem Polypeptid aufweist, das aus der Gruppe ausgewählt ist, die aus den Polypeptiden der SEQ ID Nr. 1 bis 3 besteht,
wobei das Proteinderivat davon aus der Gruppe ausgewählt ist, die aus einem biologisch aktiven Abschnitt eines "HIP/PAP"-Proteins und "HIP/PAP"-Chimären oder Fusionsproteinen besteht,
und wobei die neonatale Hirnverletzung einen pathologischen Zustand umfasst, der aus der Gruppe ausgewählt ist, die aus einer fetalen Hypoxämie, einer perinatalen Hypoxämie, einer fetalen Ischämie, einer perinatalen Ischämie und einer übermäßige Ausschüttung von exzitatorischen Neurotransmittern besteht.

2. "HIP/PAP"-Protein oder Proteinderivat davon zur Verwendung nach Anspruch 1, wobei die neonatale Hirnverletzung in einem zerebrovaskulären Unfall, der einen Schlaganfall enthält, besteht.

3. "HIP/PAP"-Protein oder Proteinderivat davon zur Verwendung nach Anspruch 1, wobei die neonatale Hirnverletzung aus der Gruppe ausgewählt ist, die aus einem ischämischen Schlaganfall und einem hämorraghischen Schlaganfall besteht.

## Revendications

1. Protéine « HIP/PAP » ou une protéine dérivée de celle-ci pour utilisation dans la prévention ou le traitement d'une lésion cérébrale néonatale, qui comprend une lésion cérébrale néonatale causée par une hypoxie du cerveau, dans laquelle la protéine « HIP/PAP » comprend une séquence d'acides aminés ayant au moins 90 % d'identité des acides aminés avec un polypeptide choisi dans le groupe constitué des polypeptides de SEQ ID N° 1 à 3, dans laquelle la protéine dérivée de celle-ci est choisie dans le groupe constitué d'une partie biologiquement active d'une protéine « HIP/PAP » et de protéines « HIP/PAP » chimériques ou de fusion,
et dans laquelle ladite lésion cérébrale néonatale englobe un état pathologique choisi dans le groupe constitué de l'hypoxémie foetale, de l'hypoxémie périnatale, de l'ischémie foetale, de l'ischémie périnatale, et d'une libération excessive de neurotransmetteurs excitateurs.

2. Protéine « HIP/PAP » ou une protéine dérivée de celle-ci pour utilisation selon la revendication 1, dans laquelle ladite lésion cérébrale néonatale consiste en un accident cérébrovasculaire, qui comprend un AVC.

3. Protéine « HIP/PAP » ou une protéine dérivée de celle-ci pour utilisation selon la revendication 1, dans laquelle ladite lésion cérébrale néonatale est choisie dans le groupe constitué de l'AVC ischémique et de l'AVC hémorragique.


### SEQUENCE LISTING

<110> ALFACT INNOVATION
<120> Novel applications of HIP/PAP or derivatives thereof
<130> W224FR
<160> 3
<170> PatentIn version 3.3
<210> 1
   <211> 138
   <212> PRT
   <213> Homo sapiens
<400> 1
   Ile Arg Cys Pro Lys Gly Ser Lys Ala Tyr Gly Ser His Cys Tyr Ala
   1               5                   10                  15
   Leu Phe Leu Ser Pro Lys Ser Trp Thr Asp Ala Asp Leu Ala Cys Gln
               20                  25                  30
   Lys Arg Pro Ser Gly Asn Leu Val Ser Val Leu Ser Gly Ala Glu Gly
           35                  40                  45
   Ser Phe Val Ser Ser Leu Val Lys Ser Ile Gly Asn Ser Tyr Ser Tyr
       50                  55                  60
   Val Trp Ile Gly Leu His Asp Pro Thr Gln Gly Thr Glu Pro Asn Gly
   65                  70                  75                  80
   Glu Gly Trp Glu Trp Ser Ser Ser Asp Val Met Asn Tyr Phe Ala Trp
                   85                  90                  95
   Glu Arg Asn Pro Ser Thr Ile Ser Ser Pro Gly His Cys Ala Ser Leu
               100                 105                 110
   Ser Arg Ser Thr Ala Phe Leu Arg Trp Lys Asp Tyr Asn Cys Asn Val
           115                 120                 125
   Arg Leu Pro Tyr Val Cys Lys Phe Thr Asp
       130                 135
<210> 2
   <211> 149
   <212> PRT
   <213> Homo sapiens
<400> 2
   Glu Glu Pro Gln Arg Glu Leu Pro Ser Ala Arg Ile Arg Cys Pro Lys
   1               5                   10                  15
   Gly Ser Lys Ala Tyr Gly Ser His Cys Tyr Ala Leu Phe Leu Ser Pro
               20                  25                  30
   Lys Ser Trp Thr Asp Ala Asp Leu Ala Cys Gln Lys Arg Pro Ser Gly
           35                  40                  45
   Asn Leu Val Ser Val Leu Ser Gly Ala Glu Gly Ser Phe Val Ser Ser
       50                  55                  60
   Leu Val Lys Ser Ile Gly Asn Ser Tyr Ser Tyr Val Trp Ile Gly Leu
   65                  70                  75                  80
   His Asp Pro Thr Gln Gly Thr Glu Pro Asn Gly Glu Gly Trp Glu Trp
                   85                  90                  95
   Ser Ser Ser Asp Val Met Asn Tyr Phe Ala Trp Glu Arg Asn Pro Ser
               100                 105                 110
   Thr Ile Ser Ser Pro Gly His Cys Ala Ser Leu Ser Arg Ser Thr Ala
           115                 120                 125
   Phe Leu Arg Trp Lys Asp Tyr Asn Cys Asn Val Arg Leu Pro Tyr Val
       130                 135                 140
   Cys Lys Phe Thr Asp
   145
<210> 3
   <211> 150
   <212> PRT
   <213> Homo sapiens
<400> 3
   Met Glu Glu Pro Gln Arg Glu Leu Pro Ser Ala Arg Ile Arg Cys Pro
   1               5                   10                  15
   Lys Gly Ser Lys Ala Tyr Gly Ser His Cys Tyr Ala Leu Phe Leu Ser
               20                  25                  30
   Pro Lys Ser Trp Thr Asp Ala Asp Leu Ala Cys Gln Lys Arg Pro Ser
           35                  40                  45
   Gly Asn Leu Val Ser Val Leu Ser Gly Ala Glu Gly Ser Phe Val Ser
       50                  55                  60
   Ser Leu Val Lys Ser Ile Gly Asn Ser Tyr Ser Tyr Val Trp Ile Gly
   65                  70                  75                  80
   Leu His Asp Pro Thr Gln Gly Thr Glu Pro Asn Gly Glu Gly Trp Glu
                   85                  90                  95
   Trp Ser Ser Ser Asp Val Met Asn Tyr Phe Ala Trp Glu Arg Asn Pro
               100                 105                 110
   Ser Thr Ile Ser Ser Pro Gly His Cys Ala Ser Leu Ser Arg Ser Thr
           115                 120                 125
   Ala Phe Leu Arg Trp Lys Asp Tyr Asn Cys Asn Val Arg Leu Pro Tyr
       130                 135                 140
   Val Cys Lys Phe Thr Asp
   145                 150
   1